**Europäisches Patentamt**

**European Patent Office**

**Office européen des brevets**

(19)

(11) Veröffentlichungsnummer : **0 149 155 B1**

(12) **EUROPÄISCHE PATENTSCHRIFT**

(45) Veröffentlichungstag der Patentschrift :
15.03.89

(51) Int. Cl.⁴ : **A 61 F 13/20**

(21) Anmeldenummer : **84115441.2**

(22) Anmeldetag : **14.12.84**

(54) Tampon für die Frauenhygiene sowie Verfahren und Einrichtung zur Herstellung desselben.

(30) Priorität : **30.12.83 DE 3347649**

(43) Veröffentlichungstag der Anmeldung :
**24.07.85 Patentblatt 85/30**

(45) Bekanntmachung des Hinweises auf die Patenterteilung : **15.03.89 Patentblatt 89/11**

(84) Benannte Vertragsstaaten :
**AT BE CH DE FR GB IT LI LU NL SE**

(56) Entgegenhaltungen :
**EP-A- 0 120 699**
**DE-A- 1 491 170**
**GB-A- 1 098 400**
**US-A- 3 523 535**
**US-A- 3 683 912**
**US-A- 4 019 226**
**US-A- 4 305 391**

(73) Patentinhaber : **JOHNSON & JOHNSON GmbH**
**Kaiserswerther Strasse 270**
**D-4000 Düsseldorf 30 (DE)**

(72) Erfinder : **Friese, Axel**
**Weststrasse 68**
**D-5600 Wuppertal (DE)**

(74) Vertreter : **Groening, Hans Wilhelm, Dipl.-Ing.**
**Patentanwälte Strehl Schübel-Hopf Groening Schulz**
**Maximilianstrasse 54 Postfach 22 14 55**
**D-8000 München 22 (DE)**

EP 0 149 155 B1

**Beschreibung**

Die Erfindung betrifft einen Tampon für die Frauenhygiene, bestehend aus einem Abschnitt bestimmter Länge eines sich aus wirr liegenden Natur- und/oder Kunststoffasern zusammensetzenden Vliesbandes mit einer der Länge des Tampons etwa entsprechenden Breite und einem nicht gewebten, thermoplastischen, an einem Ende des Vliesbandabschnitts durch Heißsiegeln befestigten Streifenabschnitt aus flüssigkeitsdurchlässigem, mindestens teilweise thermoplastischen Material, wobei der Vliesbandabschnitt im wesentlichen auf sich selbst zu einem mit einem Rückholband versehenen Tamponrohling aufgewickelt ist, auf dessen Umfang der Streifenabschnitt gerollt ist und der auf die Endform des Tampons im wesentlichen radial gepreßt ist.

Ein Tampon dieser Gattung ist aus der CH-PS 635 508 bekannt. Dieser Tampon besteht aus einer Gewebebahn, deren Breite der Länge des Tampons entspricht und die unter Bildung mehrerer Schichten gerollt ist, aus Feuchtigkeit absorbierendem Material besteht und in der ein herausragendes Rückholband verankert ist. Eine Tamponhülle aus nicht gewebtem, thermoplastischem Material ist an einem Ende der Gewebebahn durch Heißsiegeln befestigt sowie um das absorbierende Material herum gerollt und umgibt die Umfangsfläche des Tampons. Der Tampon wird nach dem Wickeln des absorbierenden Materials auf seine Endabmessung gepreßt. Die Hülle ist an dem Ende der Gewebebahn mittels eines Klebestreifens oder einer Druckverbindung unter Wärmeeinwirkung befestigt. Das zusammengerollte Gewebe aus absorbierendem Material wird durch zumindest eine Schicht des Hüllenmaterials abgedeckt, indem dieses Material um das absorbierende Material gerollt wird. Infolgedessen ist der Tampon vollständig in die Hülle eingelegt. Die über die Enden des Tampons axial vorstehenden Enden der Hülle werden in die Mitte des Tampons hineingesteckt. Die vollständige Umhüllung der aus absorbierendem Material bestehenden Rolle bietet zwar den Vorteil, mit dem Einstecken der Hüllenenden in die entsprechenden Tamponenden die Absonderung von Flusen aus dem absorbierenden Material zu verhindern. Dies geschieht jedoch bei dem bekannten Tampon auf Kosten der Sauggeschwindigkeit und Saugfähigkeit des Tampons. Da nämlich das Hüllenmaterial lose auf dem Fasermaterial aufliegt, fließt auf die Hülle des Tampons auftreffende Flüssigkeit an der Außenseite der Hülle in Richtung der Körperöffnung vorbei, weil der zwischen dem Fasermaterial und der Hülle vorhandene Zwischenraum eine sofortige Weiterleitung der Flüssigkeit in das Innere des Tampons verhindert, was mindestens zum Teil durch die fehlende Kapillarwirkung bedingt ist. Das lose Hineinstecken der überstehenden Hüllenenden in die Enden der aus absorbierendem Material bestehenden Rolle führt zu einer Überlagerung von Falten des Hüllenmaterials, das auf verengtem Raum in entsprechend dichterer Packung in den Enden der Rolle untergebracht werden muß. Diese dichtere Packung des Hüllenmaterials an dem Einführende des Tampons wirkt ebenfalls einer sofortigen Aufsaugung von auf das Einführende treffender Flüssigkeit entgegen. Diese Gestaltung des bekannten Tampons, bei dem beide Rückholbandenden aus dem Zentrum und dem Innenraum zwischen der innersten Wickelschicht der Gewebebahn aus absorbierendem Material heraustreten, dient zur Lösung der Aufgabe, die Verankerung des Rückholbandes im absorbierenden Material durch das Einlegen der Hülle in den Mittelraum zwischen den Schichten aus absorbierendem Material zu bewerkstelligen. Das Festlegen des Hüllenmaterials am Ende der Gewebebahn aus absorbierendem Material dient lediglich dazu, die Herstellung des Tampons hierdurch zu vereinfachen.

Der Erfindung liegt die Aufgabe zugrunde, einen Tampon der eingangs erwähnten bekannten Gattung dadurch weiter zu verbessern, daß er gegenüber dem Ablösen von Faserflusen geschützt ist und eine Oberflächenstruktur erhält, die ein weiches und glattes Einführen des Tampons in die Körperhöhle ermöglicht, ohne daß die Saugfähigkeit, Saugkapazität sowie das wichtige Expansionsvermögen des Tampons beeinträchtigt werden. Dabei soll der Tampon trotz dieser Verbesserung in Massenproduktion bei hoher Qualität wirtschaftlich herstellbar sein.

Die Erfindung löst diese Aufgabe dadurch, daß der flüssigkeitsdurchlässige, thermoplastische Streifenabschnitt auf einer Länge mit der Außenseite des Vliesbandabschnitts durch Heißsiegeln verbunden ist, die etwa der Länge des Umfangs des Tamponrohlings entspricht, und daß das äußere, über das Ende des Vliesbandabschnitts überstehende Ende des Streifenabschnitts mit der Außenseite eines Teils des auf den Vliesbandabschnitt aufgesiegelten, aus Bikomponentenfasern bestehenden Streifenabschnitts verschweißt ist.

Dadurch, daß der Streifenabschnitt auf einer Länge auf dem Vliesbandabschnitt aufgesiegelt ist, die etwa der Länge des Umfangs des Tamponrohlings entspricht, wird über die gesamte Umfangslänge desselben eine innige Verbindung zwischen dem flüssigkeitsdurchlässigen Streifenabschnitt und dem Fasermaterial des Vliesbandabschnitts erzielt. Durch diese innige Verbindung des Hüllenmaterials mit dem Fasermaterial des Vliesbandabschnitts auf der gesamten Umfangsfläche des Tamponrohlings bzw. des gepreßten Tampons wird erreicht, daß auf das Hüllenmaterial auftreffende Flüssigkeit sofort über die Verbindungsstellen zwischen Hüllenmaterial und dem Fasermaterial des Vliesbandes in das Innere des Tampons weitergeleitet wird. Mit anderen Worten wird dadurch die Gefahr bei einer lose auf dem Vliesbandabschnitt aufliegenden Hülle vermieden, daß die Flüssigkeit an der Außenseite des Hüllenmaterials vorbei in Richtung der Körperöffnung fließen und dadurch aus der Körperöffnung austreten kann, weil es an der unmittelbaren Haftung des Hüllenmaterials an dem Fasermaterial des Vliesbandes fehlt. Die nachweisbare Beeinträchtigung der Saugfähigkeit eines mit einem Hüllenmaterial lose umgebenen Tampons wird auf diese Weise überwunden und eine Sauggeschwindigkeit und Saugkapazität erzielt, die denjenigen von hüllenlosen Tampons in etwa entspricht. Dabei wird der Komfort beim Einführen des Tampons in die Körperhöhle durch die feste Verschweißung der Hülle mit dem Fasermaterial des Tampons noch erhöht, weil auch nur die geringste Faltenbildung und eine damit möglicherweise verbundene Reizung beim Einführen des Tampons selbst bei einer verhältnismäßig trockenen, eine erhöhte Reibung beim Einführen des Tampons hervorrufenden Körperwandung vermieden wird.

Die Schweißverbindung zwischen dem Streifenabschnitt und dem Vliesbandabschnitt ist zweckmäßig auf im Abstand voneinander angeordneten Stellen vorgesehen. Dabei können die Schweißstellen zwischen dem Streifenabschnitt und dem Vliesbandabschnitt punkt- und/oder linienförmig ausgebildet sein.

Soll der Tampon gemäß der Erfindung als Digitaltampon verwendet werden, ist es vorteilhaft, den thermoplastischen, flüssigkeitsdurchlässigen Streifenabschnitt schmaler als die Breite des Vliesbandabschnitts zu bemessen, aber den das Rückholende des Tampons bildenden Längsrand desselben dabei mit dem Streifenabschnitt zu überdecken. Dabei kann der das Einführende bildende Längsrand des Vliesbandabschnitts über den zugehörigen Rand des flüssigkeitsdurchlässigen thermoplastischen Streifenabschnitts auf einer Breite vorstehen, die etwa der Höhe einer Verjüngung des Tampons an dessen Einführende entspricht. Damit ist eine sofortige Aufsaugung von Flüssigkeit bei Ingebrauchnahme des Tampons sichergestellt.

Das ungewebte Material des Streifenabschnitts enthält vorteilhaft Bikomponentenfasern, deren Ummantelung einen niedrigeren Schmelzpunkt als der Faserkern aufweist, so daß auch bei einer hohen, für die Massenproduktion erforderlichen Herstellungsgeschwindigkeit einerseits mit Sicherheit eine Versiegelung des Streifenabschnitts mit dem Vliesbandabschnitt erreicht wird, andererseits aber die Struktur des nicht gewebten Streifenabschnitts aufrechterhalten wird.

Zur Herstellung des Tampons geht die Erfindung von einem Verfahren aus, das aus der bereits genannten CH-PS 635 508 bekannt ist und bei dem ein Längenabschnitt eines flüssigkeitsdurchlässigen, thermoplastischen, ungewebten Materialstreifens auf die Außenseite des rückwärtigen Endes des Vliesbandabschnitts bestimmter Länge aufgeschweißt wird, dessen Breite der Länge des Tampons etwa entspricht, woraufhin der Vliesbandabschnitt mit einem Rückholband versehen und auf sich selbst zur Bildung eines Tamponrohlings aufgerollt wird, so daß der Streifenabschnitt sich über den Umfang des Tamponrohlings erstreckt, welcher anschließend auf die Endform des Tampons gepreßt wird.

Dieses Verfahren ist gemäß der Erfindung dadurch gekennzeichnet, daß der nicht gewebte thermoplastische, sich aus Bikomponenten zusammensetzende Streifenabschnitt auf einer der Umfangslänge des Tamponrohlings etwa entsprechenden Länge auf den Vliesbandabschnitt aufgesiegelt wird und nach dem Aufrollen des Vliesbandabschnitts zu dem Tamponrohling das über den Vliesbandabschnitt hinausragende unversiegelte Ende mit dem Streifenabschnitt unter Anwendung von Hitze und Druck verschweißt wird.

Die Ansprüche 8 bis 13 stellen zweckmäßige Weiterbildungen dieses Verfahrens dar.

Bei Herstellung eines Digitaltampons gemäß der Erfindung empfiehlt sich die Verwendung eines flüssigkeitsdurchlässigen thermoplastischen Streifens, der schmaler als das Vliesband ist und der sich von dem das Rückholende des Tampons bildenden Längsrand des Vliesbandes nur bis in die Nähe des das Einführende des Tampons bildenden Längsrandes des Vliesbandes erstreckt. Dadurch ist es möglich, das unbedeckte Einführende des Tampons nach dem radialen Pressen des Tamponrohlings kugelkalottenartig zu verformen und damit ein besseres Einführen und sofortiges Absorbieren von Flüssigkeit zu ermöglichen.

Eine Einrichtung zur Herstellung des erfindungsgemäßen Tampons besteht zweckmäßig aus folgenden, in Verarbeitungsrichtung hintereinandergeschalteten, im Oberbegriff des Patentanspruchs 14 als zum Stand der Technik gerechneten Vorrichtungen, die z. B. aus den DE-PSen 1 264 686 oder 2 253 180 im wesentlichen bekannt sind : einer Vorratsrolle für ein fortlaufendes Vliesband aus Naturund/oder Kunststoffasern ; einer Schwächungsstation für das Vliesband ; einer Transportvorrichtung für das Vliesband ; einer Vliesband-Trennvorrichtung zum fortlaufenden Abtrennen von Vliesbandabschnitten ; einer Rückholband-Anbring- und -Knotvorrichtung ; einer Wickelstation zum Aufrollen des Vliesbandabschnitts unter Bildung eines Tamponrohlings ; einer etwa in der Transportebene des Vliesbandes hin- und herbewegbaren Transport- und Trennzange zum Abtrennen je eines Vliesbandabschnitts, und einer Vorrichtung zum Überführen des Tamponrohlings zu einer Presse, in welcher der Tamponrohling auf die Endform des Tampons im wesentlichen radial preßbar ist, sowie einer Formvorrichtung für das Einführende des Tampons.

Diese Einrichtung ist gemäß der Erfindung dadurch gekennzeichnet, daß eine Vorratsrolle für einen fortlaufenden Streifen aus nicht gewebtem, flüssigkeitsdurchlässigem, thermoplastischem Material von einer gegenüber dem Vliesband geringeren Breite vorgesehen ist, welcher eine Schneidstation zum überwiegenden Durchtrennen des fortlaufenden Streifens in Abschnitte bestimmter Länge nachgeschaltet ist, hinter der eine Abtrenn- und Übergabevorrichtung für die Streifenabschnitte angeordnet ist, der eine Siegelstation zum Aufsiegeln des thermoplastischen Streifenabschnitts auf die Außenseite des Vliesbandes nachgeschaltet ist, welcher eine Vorrichtung für den kontinuierlichen Transport des Vliesbandes mit dem darauf aufgesiegelten Streifenabschnitt zu der Vliesband-Trennvorrichtung nachgeordnet ist, und daß der Wickelstation eine beheizbare Schweiß- und Glättvorrichtung zum Andrücken des sich über das rückwärtige Ende des Vliesbandabschnitts frei und unversiegelt erstreckenden Endes des Streifenabschnitts gegen den auf den Vliesbandabschnitt aufgesiegelten Streifenabschnitt zugeordnet ist.

Die Ansprüche 15 bis 33 betreffen zweckmäßige Weiterbildungen der erfindungsgemäßen Einrichtung.

Dadurch, daß das flüssigkeitsdurchlässige thermoplastische Material auf dem Vliesband befestigt ist, kann sich kein Zwischenraum zwischen dem Vliesbandabschnitt und dem Streifenabschnitt bilden. Dabei bleiben die Expansionsfähigkeit und die Saugkapazität des gepreßten Tampons voll erhalten. Das thermoplastische Material am Umfang des Tampons setzt dem Einführen des Tampons in die Körperhöhle einen wesentlich geringeren Widerstand entgegen, so daß das Einführen für die Benutzerin angenehmer und leichter vonstatten geht, während gleichzeitig die Ablösung von Fasern beim Einführen und Entfernen des Tampons ausgeschlossen ist. Die feste Verbindung des thermoplastischen Streifenabschnitts mit dem Vliesbandabschnitt gewährleistet eine einwandfreie, glatte Konturierung der Außenseite des Tampons, was ebenfalls zu einem erhöhten Komfort beim Einführen des Tampons beiträgt. Dabei hat sich überraschend gezeigt, daß auch bei sehr hohen Produktionsgeschwindigkeiten eine einwandfreie Versiegelung des thermoplastischen Streifenabschnitts unter Beibehaltung seiner Struktur mit dem Vliesband möglich ist. Daher bleibt der nicht gewebte Streifenabschnitt, wie nicht ohne weiteres zu erwarten war, auch am Vliesbandabschnitt beim Aufrollen desselben zu dem Wickelrohling fest haften, obwohl Fliehkräfte auf ihn und insbesondere auf das freie unversiegelte Ende desselben einwirken. Infolge der festen Siegelverbindung zwischen dem Streifenabschnitt und dem Vliesband behält das thermoplastische Material seine glatte Oberfläche bei, wenn der expandierte und mit aufgesaugter Körperflüssigkeit gefüllte Tampon aus der Körperhöhle mit Hilfe des Rückholbandes wieder entfernt wird. Die mit dem Streifenabschnitt erreichte geringere Reibung gegenüber der Wandung der Körperhöhle trägt dazu bei, daß der Faserverband des expandierten Tampons seine dem Tamponrohling etwa entsprechende Form beibehält.

Die Erfindung ist nachstehend anhand der schematischen Zeichnung eines Ausführungsbeispiels näher beschrieben. Es zeigen :

Fig. 1 bis 6 Verfahrensschritte zur Herstellung des erfindungsgemäßen Tampons und

Fig. 7 eine Einrichtung zum Herstellen des erfindungsgemäßen Tampons in schaubildlicher Ansicht und

Fig. 8 eine abgeänderte Ausführungsform der Einrichtung in Fig. 7 und

Fig. 9 eine Draufsicht auf eine Transportund Trennzange in der Greifstellung.

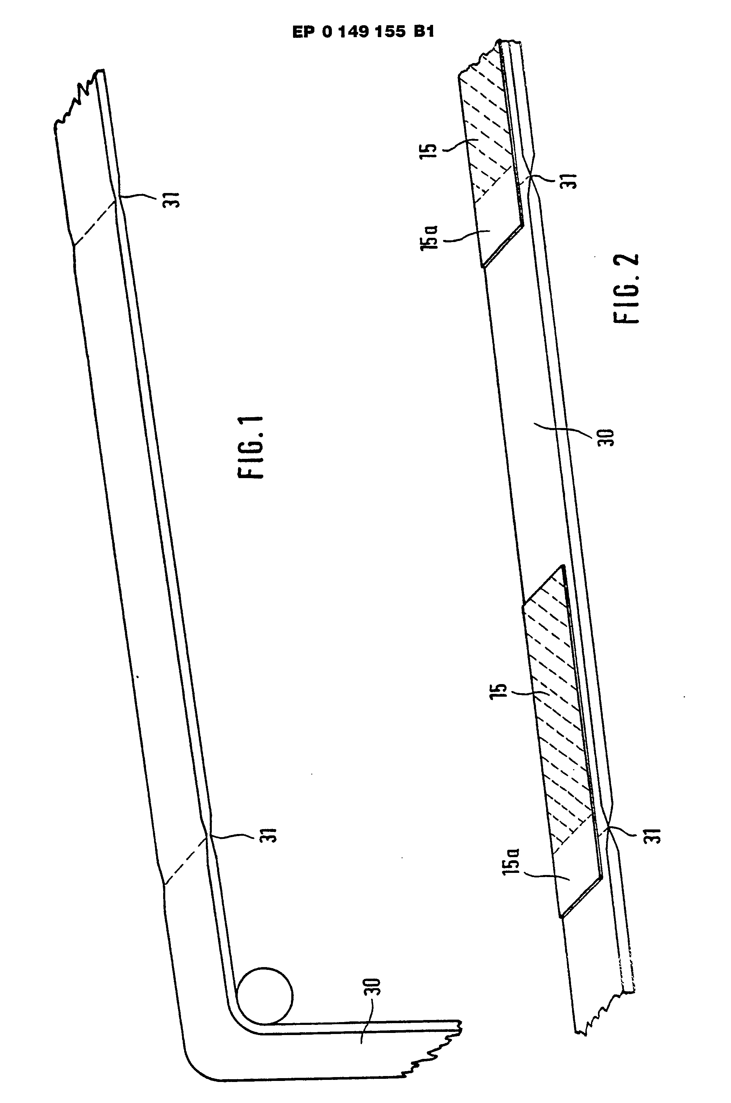

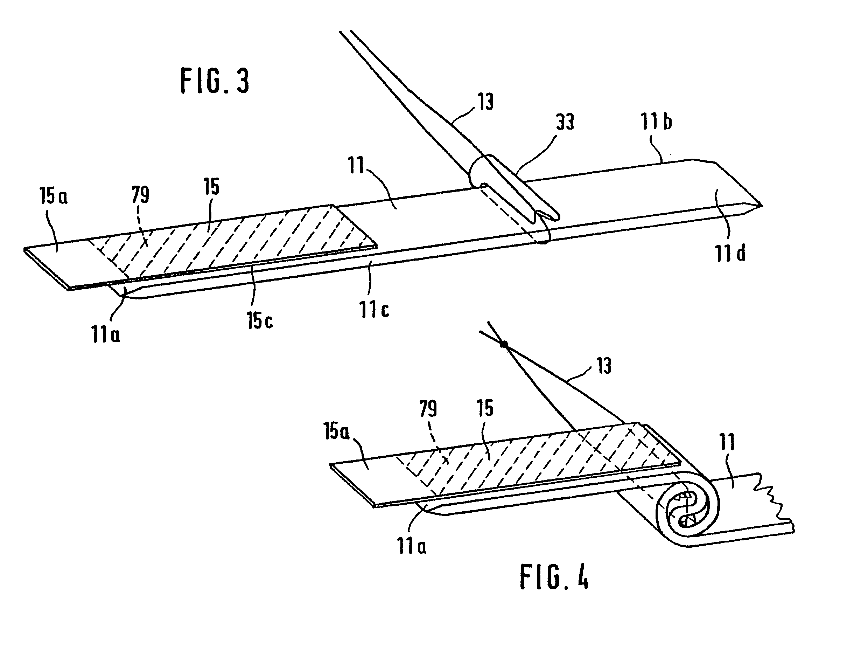

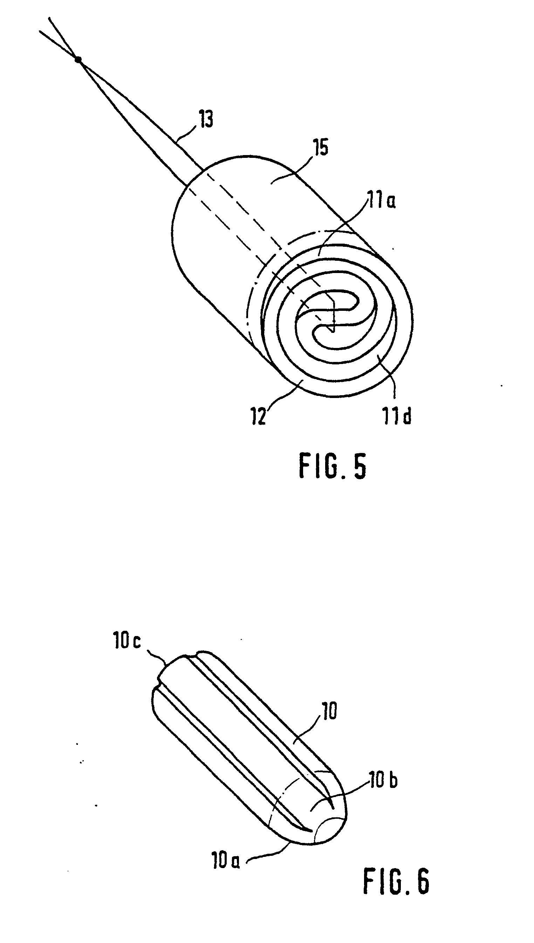

In Fig. 6 ist ein Tampon 10 für die Frauenhygiene abgebildet, der aus einem aus Fig. 3 ersichtlichen Abschnitt 11 bestimmter Länge eines sich aus wirr liegenden Natur- und/oder Kunststofffasern zusammensetzenden kalandrierten Vliesbandes 30 (Fig. 1, 2, 7 und 8) mit einer der Länge des Tampons 10 etwa entsprechenden Breite besteht. Dieser Vliesbandabschnitt 11 ist um eine quer zu seiner Längsrichtung außerhalb der Längsmitte des Vliesbandabschnitts 11 liegende Achse zu einem Tamponrohling 12 im wesentlichen auf sich selbst aufgerollt und gleichzeitig mit einem Rückholband 13 versehen. Der Tamponrohling 12 ist auf die Endform des Tampons 10 im wesentlichen radial zur Wickelachse gepreßt. Durch die Pressung ist der Tampon mit über gleiche Umfangswinkel verteilt angeordneten Preßkerben versehen. Das Einführende 10a des Tampons 10 ist als rundkuppenartige Verjüngung 10b ausgebildet.

Gemäß der Erfindung ist auf den die Umfangsfläche des Tampons 10 bildenden Teil des Vliesbandabschnitts 11 ein Streifenabschnitt 15 aus flüssigkeitsdurchlässigem thermoplastischen Material unter Anwendung von Hitze und Druck aufgesiegelt, der aber länger als der Umfang des Tamponrohlings 12 bemessen ist.

Fig. 2 bis 4 zeigen, daß der thermoplastische Streifenabschnitt 15 mit dem Vliesband 30 mittels im Abstand paralleler Schweißlinien 79 fest verbunden ist, die mit der Längsrichtung des Vliesbandes einen spitzen Winkel bilden. Die Abstände zwischen den einzelnen Schweißlinien 79 sind so bemessen, daß zwischen den Schweißlinien das flüssigkeitsdurchlässige Material des Streifenabschnittes 15 dicht auf dem Vliesbandmaterial aufliegt. Dadurch ist gewährleistet, daß Flüssigkeit, die auf den Streifenabschnitt 15 auf der Umfangsfläche des Tampons auftrifft, durch die Kapillarwirkung des Fasermaterials des darunterliegenden Vliesbandabschnittes 11 sofort in das Innere des Tampons angesaugt wird.

Das äußere Ende 15a des Streifenabschnitts 15 erstreckt sich über das äußere Ende 11a des Vliesbandabschnitts 11 hinaus und ist mit der Außenseite eines Teils des auf den Vliesbandabschnitt 11 aufgesiegelten Streifenabschnitts 15 unter Anwendung von Hitze und Druck verschweißt.

Es ist ersichtlich, daß der thermoplastische, flüssigkeitsdurchlässige Streifenabschnitt 15 schmaler als die Breite des Vliesbandabschnitts 11 ist, aber mit dem das Rückholende 10c des Tampons 10 bildenden Längsrand 11b des Vliesbandabschnitts 11 bündig ist. Der das Einführende 10a des Tampons 10 bildende Längsrand 11c des Vliesbandabschnitts 11 steht über den zugehörigen Rand 15c des flüssigkeitsdurchlässigen thermoplastischen Streifenabschnitts 15 auf einer Breite vor, die etwa der Höhe der rundkuppenartigen Verjüngung 10b des Tampons 10 am Einführrende 10a entspricht. Der flüssigkeitsdurchlässige Streifen setzt sich zumindest vorwiegend aus thermoplastischem, nicht gewebten Fasermaterial zusammen, welches vorzugsweise aus einer Bikomponentenfaser hergestellt ist, deren Komponenten z. B. aus einem Polyesterkern und einer HD-Polyäthylen-Ummantelung bestehen. Es ist besonders zweckmäßig, wenn das Hochdruck-Polyäthylen einen niedrigeren Schmelzpunkt als der Polyester aufweist. Da das freie äußere Ende 15a des thermoplastischen Streifenabschnitts 15 mit der Außenseite desjenigen Längenabschnitts des Streifenabschnitts 15 verschweißt ist, der auf den Vliesbandabschnitt 11 aufgesiegelt ist, bildet der Streifenabschnitt 15 in Umfangsrichtung des Tamponrohlings 12 eine mit diesem fest verbundene Hülle, deren Außendurchmesser demjenigen des Tamponrohlings 12 entspricht. Da die Hülle mit dem Fasermaterial des Vliesbandes 30 fest verschweißt ist, wird die hohe Kapillarwirkung des Fasermaterials durch die Hülle hindurch sichergestellt, so daß der Tampon 10 nach dem Pressen in einer mit sternförmig angeordneten Backen versehenen Presse, wie sie z. B. in der DE-PS 1 491 161 beschrieben ist, eine Saugkapazität und Saugleistung aufweist, die einem entsprechenden Tampon ohne Umhüllung ebenbürtig ist, aber gegen die Ablösung von Fasern und Entspiralisieren geschützt ist.

Das Verfahren zum Herstellen des vorstehend beschriebenen Tampons besteht aus folgenden Schritten, die anhand der Fig. 1 bis 6 nachstehend beschrieben werden :

Gemäß Fig. 1 wird ein kalandriertes Vliesband 30, das aus wirr liegenden Natur- und/oder Kunststoffasern besteht und eine der Länge des Tampons 10 entsprechende Breite aufweist, kontinuierlich zugeführt und zwischen Abschnitten 11 einer für die Herstellung des Tampons 10 notwendigen Länge quer zu seiner Längsrichtung jeweils durch sogenannte Schwachstellen 31 z. B. durch Perforieren geschwächt. Diese Schwächung wird zusätzlich durch ein Strecken des Vliesbandes 30 erreicht, so daß in der Streckzone oder Schwachstelle 31 eine Verdünnung des Vliesbandes 30 bzw. eine Verminderung seines Querschnitts, nicht aber eine Durchtrennung des Vliesbandes 30 eintritt. Das Vliesband wird kontinuierlich in Richtung des Pfeiles x weiterbewegt. Etwa gleichzeitig wird ein fortlaufend zugeführter Streifen 32 aus thermoplastischem, flüssigkeitsdurchlässigem, nicht gewebtem Material jeweils in einen Streifenabschnitt 15 durchtrennt, dessen Länge den Umfang des in Fig. 5 gezeigten Tamponrohlings 12 überschreitet. Danach wird der flüssigkeitsdurchlässige, thermoplastische Streifenabschnitt 15 auf die Außenseite eines in Bewegungsrichtung x des Vliesbandes 30 rückwärtigen, vor einer Schwachstelle 31 liegenden Bereiches des Vliesbandes 30 unter Ausübung von Hitze und Druck entlang paralleler, schräg zur Längsrichtung des Vliesbandes 30 verlaufender Schweißlinien 79 aufgesiegelt. Die Anordnung des Streifennabschnitts 15 auf der Oberseite des Vliesbandes 30 ist dabei derart vorgesehen, daß sich das in Bewegungsrichtung x des Vliesbandes 30 rückwärtige äußere Ende 15a (Fig. 3) des Streifenabschnitts 15 frei, d. h. unversiegelt, über die Schwachstelle 31 des Vliesbandes 30 hinaus erstreckt. Danach wird das Vliesband 30 an der Schwachstelle 31 durchtrennt, so daß der Vliesbandabschnitt 11 entsteht.

Danach wird der Vliesbandabschnitt 11 um eine quer zu seiner Längsrichtung verlaufende Achse, die in Fig. 3 durch einen Wickeldorn 33 dargestellt ist, gemäß Fig. 4 im wesentlichen auf sich selbst zu einem Tamponrohling 12 aufgerollt, der in Fig. 5 gezeigt ist. Dieses Aufrollen des Vliesbandabschnitts 11 geschieht in der Weise, daß das eine Ende 11a der äußeren Schicht des aufgerollten Vliesbandabschnitts 11 das andere Ende 11d (siehe Fig. 3 und 5) der darunter liegenden Schicht des aufgerollten Vliesbandabschnitts 11 in Umfangsrichtung des Tamponrohlings 12 überlappt. Hierdurch wird am Außenumfang des Tamponrohlings 12 eine gleichmäßigere Materialverteilung und infolgedessen eine im wesentlichen zylindrische Form desselben erreicht.

Wie aus Fig. 5 ersichtlich ist, ist die Länge des thermoplastischen, flüssigkeitsdurchlässigen Streifenabschnitts 15 so bemessen, daß dieser den Umfang des Tamponrohlings 12 vollständig auf der vorgesehenen Breite umschließt, wobei zunächst noch das freie bzw. unversiegelte Ende 15a an der Außenseite vorsteht. Der Wickelvorgang wird nunmehr dadurch abgeschlossen, daß dieses freie, unversiegelte Ende 15a des Streifenabschnitts 15 unter Ausübung von Hitze und Druck an dem dem äußeren Ende des Vliesbandabschnitts 11 in Umfangsrichtung des Tamponrohlings 12 benachbarten Teil des auf den Vliesbandabschnitt 11 aufgesiegelten thermoplastischen Streifenabschnitts 15 aufgeschweißt wird. Da hierbei die Oberflächen aus HD-Polyäthylen der Schmelzfasern aufeinander zu liegen kommen, braucht der Siegeldruck nicht so groß zu sein, wie beim vorherigen Aufsiegeln des Streifenabschnitts 15 auf das Vliesband 30, um eine gute Versiegelung zu erreichen.

Gemäß Fig. 3 wird vor dem Wickeln das Rückholband 13 quer zur Längsrichtung des Vliesbandabschnitts 11 um dieses herum gelegt und gegebenenfalls anschließend an seinen freien Enden verknotet. Der fertiggestellte Tamponrohling 12 wird anschließend einer Presse zugeführt, die gemäß der DE-PS 1 491 161 aus sternförmig angeordneten Preßbacken besteht, in der der Tamponrohling 12 im wesentlichen radial auf die Endform des in Fig. 6 dargestellten Tampons 10 gepreßt wird. Danach wird das Einführende 10a des Tampons in Form einer rundkuppenartigen Verjüngung 10b verformt, um das Einführen des Tampons in die Körperhöhle zu erleichtern. Es ist für das vorliegende Verfahren nicht wesentlich, wo und wie das Rückholband 13 im einzelnen appliziert wird.

Es versteht sich, daß die Länge des Streifenabschnitts 15 von dem Enddurchmesser des Tamponrohlings 12 abhängt. Danach richtet sich auch die Länge des frei überstehenden, unversiegelten Endes 15a des Streifenabschnitts 15, die in der Regel zwischen 10 und 50 mm beträgt.

Aus den Fig. 2 bis 6 ist ersichtlich, daß die Außenseite des Vliesbandes 30 mit dem flüssigkeitsdurchlässigen, thermoplastischen Streifenabschnitt 15 auf einer Breite abgedeckt wird, die sich von dem das Rückholende 10c des Tampons 10 bildenden Längsrand 11b des Vliesbandes 30 nur bis in die Nähe des das Einführende 10a des Tampons 10 bildenden Längsrandes 11c des Vliesbandabschnitts 11 erstreckt. Der vom thermoplastischen Streifenabschnitt 15 unbedeckte Längsrand 11c des Vliesbandes 30 ist so breit bemessen, daß er zur Ausformung der kugelkalottenartigen Verjüngung 10b des Einführendes 10a des Tampons 10 nach dem Pressen des Tamponrohlings 12 in die Endform des Tampons 10 verformt werden kann. Dadurch ist gleichzeitig gewährleistet, daß das von dem Streifenabschnitt 15 freie Einführende 10a unmittelbar von der aufzusaugenden Körperflüssigkeit beaufschlagt wird und der Tampon infolgedessen unverzögert expandiert und dadurch seine volle Saugfähigkeit entfalten sowie die volle Schutzfunktion für die Benutzerin übernehmen kann. Dabei vermittelt das thermoplastische, nicht gewebte Material infolge seines geringen Reibungskoeffizienten die Annehmlichkeit des leichten, weichen Einführens des Tampons in die Körperhöhle sowie einen Schutz gegenüber einem Ablösen von Fasern beim Einführen bzw. Entfernen des Tampons in die bzw. aus der Körperhöhle. Infolge der im Vergleich zu dem Vliesbandmaterial wesentlich höheren Gleitfähigkeit des nicht gewebten Materials des Streifenabschnitts 15 am Umfang des Tampons wird ferner einer etwaigen Neigung zum Entspiralisieren des Tampons beim Entfernen des Tampons aus der Körperhöhle am Ende seines Gebrauchs wesentlich entgegengewirkt.

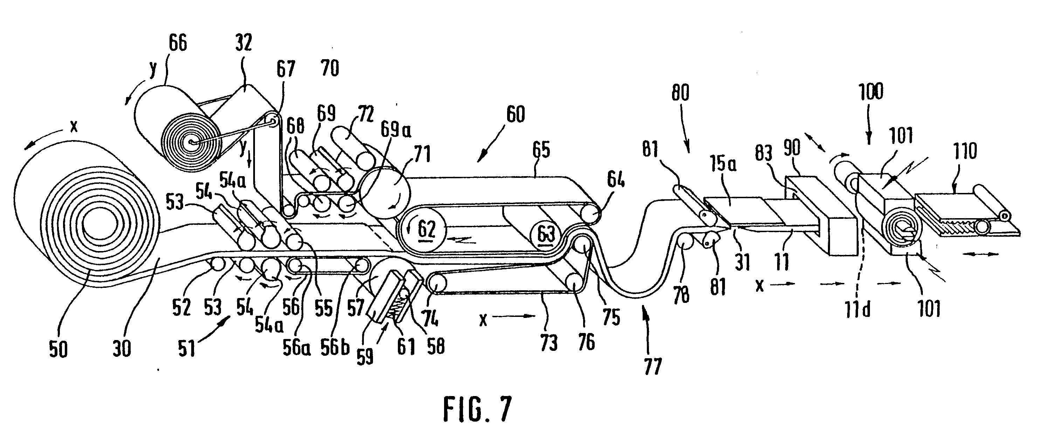

Fig. 7 zeigt eine Einrichtung zum Herstellen des erfindungsgemäßen Tampons unter Anwendung des vorstehend beschriebenen Verfahrens. Im linken Teil der Zeichnung ist ein Vliesband 30 dargestellt, das von einer Vorratsrolle 50 kontinuierlich in Bewegungsrichtung x einer Schwächungsstation 51 zugeführt wird. Die Schwächungsstation 51, der eine Stützrolle 52 vorgeschaltet ist, besteht in Bewegungsrichtung des Vliesbandes 30 aus einem Paar Perforier- und Klemmwalzen 53 sowie einem Paar Streckwalzen 54. Da die Perforier- und Klemmwalzen 53 das Vliesband vor den Streckwalzen 54 im Augenblick der Streckung festhalten und die Streckwalzen 54 mit ihren Streckbacken 54a eine Beschleunigung des von ihnen erfaßten Vliesbandes in Transportrichtung x hervorrufen, wird auf der Strecke zwischen den Perforier- und Klemmwalzen 53 und den Streckwalzen 54 das Vliesband 30 ausgedünnt bzw. im Querschnitt vermindert, so daß die Schwachstelle 31 entsteht. Der Schwächungsstation 51 sind an der Oberseite der Bewegungsbahn des Vliesbandes eine Transportwalze 55 und an der Unterseite ein endloses Tragband 56 nachgeschaltet, das um eine vordere, unmittelbar unter der Transportwalze 55 liegende Antriebs- und Spannrolle 56a und eine weitere Umlenkrolle 56b herumgeführt ist.

Eine Siegelstation 60 besteht aus einer unter der Bewegungsbahn des Vliesbandes 30 angeordneten Druckrolle 57, deren Achse 58 an beiden Enden in einer auf und ab bewegbaren Führung 59 gelagert und auf je einer Druckfeder 61 abgestützt ist. Die federbelastete Druckrolle 57 ist mittels der Führung 59 in Richtung einer Siegelrolle 62 intermittierend, z. B. mittels einer nicht gezeigten Steuerkurve, bewegbar. Die Siegelrolle 62 ist in geeigneter Weise, z. B. mittels elektrischer Widerstandsheizung, beheizbar und entgegen dem Uhrzeigersinn antreibbar. Der Siegelrolle 62 sind darüber hinaus eine größere und eine kleinere Umlenkrolle 63 und 64 zugeordnet. Die kleinere Umlenkrolle 64 ist in Bewegungsrichtung x des Vliesbandes 30 hinter der Umlenkrolle 63 größeren Durchmessers erhöht angeordnet. Um die Siegelrolle 62 und die beiden Umlenkrollen 63 und 64 ist ein endloses Siegelband 65 gelegt, das aus einem Material hergestellt ist, welches gegenüber der Temperatur der Siegelrolle 62 hitzebeständig, aber in der Lage ist, die Wärme der Siegelrolle 62 auf die Außenseite des endlosen Siegelbandes 65 zu übertragen. Bevorzugt werden als Werkstoffe für das Siegelband 65 Silicongummi oder Bandstahl verwendet, die eine gute Wärmeübertragung von der Siegelrolle 62 auf den thermoplastischen Streifenabschnitt 15 und eine hohe Lebensdauer gewährleisten. Die Oberfläche des Siegelbandes 65 kann entsprechend einem gewünschten Siegelmuster profiliert sein, so daß die Schweißstellen zwischen Vliesband 30 und Streifenabschnitt 15 im Abstand voneinander vorgesehen sind (Schweißlinien 79 in Fig. 3).

Es ist ersichtlich, daß vor der Siegelstation 60 oberhalb des Vliesbandes 30 und der Schwächungsstation 51 eine Vorratsrolle 66 für den fortlaufenden Streifen 32 aus nicht gewebtem, flüssigkeitsdurchlässigem und thermoplastischem Fasermaterial vorgesehen ist, der über eine abgefederte Umlenkrolle 67 einer Schneidstation 70 zugeführt wird.

Die Schneidstation 70 besteht aus einem Paar oberhalb und unterhalb des Streifens 32 angeordneter, gegenläufig angetriebener Transportwalzen 68 und einem danach angeordneten Paar Schneidwalzen 69, 69a die ebenfalls gegenläufig angetrieben werden und von denen die Messerwalze 69 auf einem wesentlichen Teil einer Mantellinie mit Schneiden für die überwiegende Durchtrennung des Streifens 15 versehen ist. Die Umfangsgeschwindigkeit der Transportwalzen 68 und Schneidwalzen 69, 69a entspricht etwa der halben Transportgeschwindigkeit des Vliesbandes 30, derart, daß der Streifen 32 in Richtung des Pfeiles y von der Vorratsrolle 66 mit der der Länge des zu applizierenden Streifenabschnitts 15 des nicht gewebten Materials entsprechenden Geschwindigkeit fortlaufend abgezogen werden kann.

Die Schneidwalzen 69 durchschneiden den Streifen 32 in Querrichtung im wesentlichen, jedoch nicht vollständig, so daß der vorlaufende, durch das Schneiden gebildete Streifenabschnitt 15 mit dem nachfolgenden Streifen noch über einige wenige sog. Stege verbunden ist.

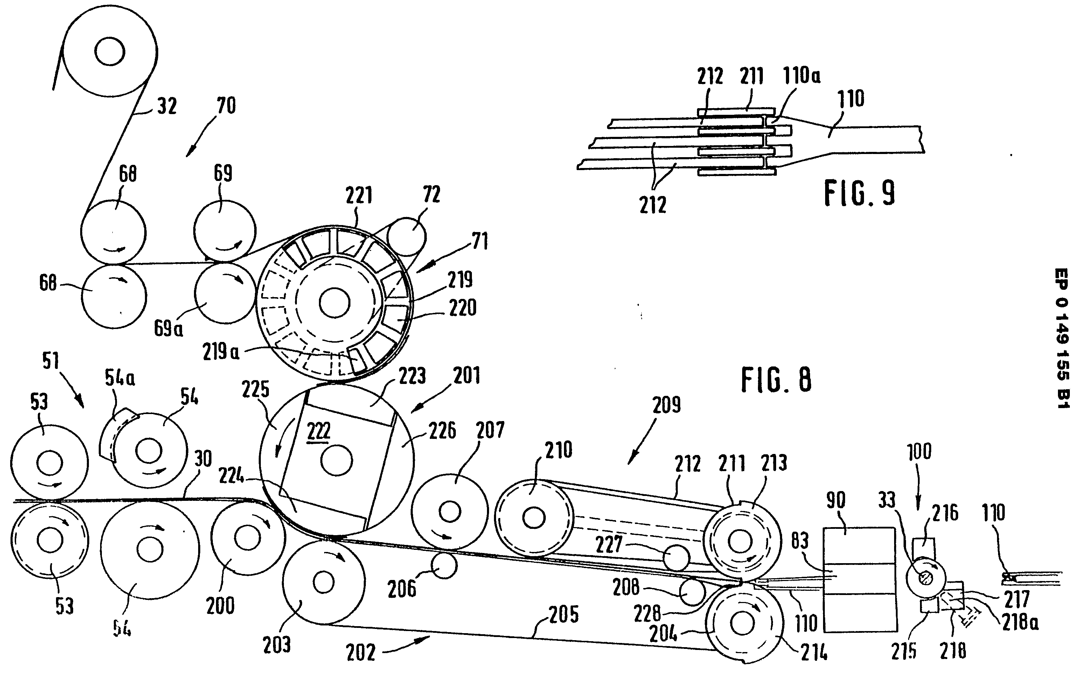

Der Schneidstation 70 ist eine Vakuumrolle 71 nachgeschaltet, deren konstruktiver Aufbau in Fig. 8 näher gezeigt und im Zusammenhang mit der Beschreibung der darin gezeigten abgeänderten Ausführungsform weiter unten näher beschrieben ist. Der Vakuumrolle 71 ist eine Beschleunigungswalze 72 kleineren Durchmessers an der Oberseite zugeordnet und dient dazu, das nicht gewebte Material gegen die Vakuumrolle 71 anzudrücken. Die Umfangsgeschwindigkeit der Vakuumrolle 71 und der Beschleunigungswalze 72 entspricht der Transportgeschwindigkeit des Vliesbandes 30. Die Vakuumrolle 71 ist auf ihrer Umfangsfläche mit Löchern versehen, wobei der innere Hohlraum der Vakuumrolle an eine Vakuumquelle angeschlossen ist, die ein- und abschaltbar ist (nicht gezeigt). Daher kann der von der Schneidstation 70 kommende Streifenabschnitt 15 gegen den Umfang der Vakuumrolle 71 angesaugt und in gestreckter Lage in Uhrzeigerrichtung in den Spalt mitgenommen werden, den die Vakuumrolle 71 mit der Beschleunigungswalze 72 bildet. Sobald das vordere Ende des Streifenabschnitts 15 in den Spalt zwischen der Beschleunigungswalze 72 und der Vakuumrolle 71 gerät, wird der Streifenabschnitt 15 auf die doppelte Geschwindigkeit, nämlich die Vliesbandgeschwindigkeit, beschleunigt und infolgedessen vollständig von dem nachfolgenden, nicht gewebten Streifen 32 im Bereich der Schnittstelle abgerissen, die in der Schneidstation 70 hergestellt wurde.

Die Vakuumrolle 71 nimmt nunmehr den auf ihrem Umfang angesaugten Streifenabschnitt 15 zu dem Spalt mit, den die Vakuumrolle 71 mit dem endlosen Siegelband 65 auf der Siegelrolle 62 bildet. Da die Vakuumrolle 71 schräg oberhalb vor der Siegelrolle 62 das endlose Siegelband 65 berührt und an dieser Stelle das Vakuum in Drehrichtung der Vakuumrolle 71, wie weiter unten beschrieben, abgesperrt wird, wird der thermoplastische Streifenabschnitt 15 zum frühestmöglichen Zeitpunkt durch die Siegelrolle 62 und das erhitzte endlose Siegelband 65 durch Adhäsion mitgenommen und erwärmt. Infolgedessen ist der Streifenabschnitt 15 bei der weiteren Mitnahme durch den Spalt zwischen Siegelrolle 62 und Vakuumrolle 71 durch das endlose Siegelband 65 beim Auftreffen auf die Oberfläche des Vliesbandes 30 soweit vorgewärmt, daß die Schmelzfasern des Streifenabschnitts 15 im Bereich des Rollenspalts zwischen der Druckrolle 57 und der Siegelrolle 62 schmelzen und infolge der richtigen Druck- und Temperatureinstellung eine innige Verbindung mit den Fasern an der Oberfläche des Vliesbandes hergestellt wird.

Im Bereich des oberen Siegelbandes 65 befindet sich unterhalb der Bewegungsbahn des Vliesbandes 30 wiederum ein endloses Transportband 73, das um eine in Bewegungsrichtung x des Vliesbandes vordere Umlenkrolle 74 geführt ist, die hinter der Druckrolle 57 sowie in geringem Abstand unterhalb des Vliesbandes 30 angeordnet ist. Das Transportband 73 drückt das Vliesband mit dem darauf aufgesiegelten Streifenabschnitt 15 des nicht gewebten Materials gegen die größere Umlenkrolle 63 des Siegelbandes 65, da eine Umlenkrolle 75 des unteren endlosen Transportbandes 73 in den Walzenspalt zwischen der größeren Umlenkrolle 63 und der dahinter, aber höher liegenden kleineren Umlenkrolle 64 des Siegelbandes 65 hineingedrückt wird. Das Transportband 73 wird etwa quer zum Vliesband 30 nach unten um eine weitere Umlenk- oder Spannrolle 76 herumgeführt. Die endlosen Bänder 65 und 73 werden kontinuierlich im bzw. entgegen dem Uhrzeigersinn angetrieben, so daß das Vliesband mit dem darauf aufgesiegelten thermoplastischen Streifenabschnitt 15 ständig weitertransportiert wird. Durch die beschriebene Umlenkung wird eine einwandfreie Ablösung des Vliesbandabschnitts 11 mit dem darauf aufgesiegelten Streifenabschnitt von dem Siegelband 65 sichergestellt. Gegebenenfalls können die Umlenkrollen 63, 64 gekühlt werden.

Fig. 2 zeigt das Vliesband 30 nach dem Aufsiegeln des Streifenabschnitts 15 auf das jeweils rückwärtige Ende 11a eines Vliesbandabschnitts 11. Es ist ersichtlich, daß das rückwärtige Ende 15a des Streifenabschnitts 15 sich über die Schwachstelle 31 des Vliesbandes 30 hinaus bis zu dem nachfolgenden Vliesbandabschnitt 11 erstreckt, aber nicht mit dem Vliesband 30 versiegelt ist. Um dies zu gewährleisten, ist die Führung 59 mit der federbelasteten Druckrolle 57 in und entgegen der Siegelrolle 62 auf und ab bewegbar ausgebildet und so steuerbar, daß die Druckrolle 57 in dem in Fig. 7 dargestellten Betriebszustand nach unten aus dem Berührungszustand mit dem Vliesband 30 wegbewegt wird. Dadurch hängt das Vliesband 30 zwischen den Transportbändern 56 und 73 durch und wird auf dem Transportband 73 abgestützt, dessen Umlenkrolle 74 gegenüber der Bewegungsbahn des Vliesbandes 30 tiefer liegt, wenn dieses mittels der Druckrolle 57 gegen die Siegelrolle 62 gepreßt wird. Im Endbereich des endlosen Siegelbandes 65 ist dessen Temperatur so gering, daß eine Erweichung oder ein Aufschmelzen der Schmelzfasern des freien, unversiegelten Endes 15a des Streifenabschnitts 15 ausgeschlossen ist und dieses weiterhin unversiegelt auf dem Vliesband 30 aufliegt.

An die beiden endlosen Bänder 65 und 73 schließt sich ein Ausgleichs- oder Entspannungsbereich 77 für das Vliesband 30 mit den darauf in Längenabständen aufgeschweißten Streifenabschnitten 15 an, in dem das Vliesband zwischen den beiden genannten endlosen Transportbändern und einer Führungsrolle 78 durchhängt. Dieser Ausgleichsbereich trennt den kontinuierlichen vorderen Arbeitsprozeß von dem nachfolgenden intermittierend arbeitenden Prozeß.

An die Führungsrolle 78 schließt sich ein Teil einer Vliesbandtrennvorrichtung 80 an, der aus einem Paar Klemmbacken 81 besteht, die jeweils an der Ober- und Unterseite der Bewegungsbahn des Vliesbandes 30 hin und her schwenkbar angebracht sind. Die Klemmbacken 81 klemmen intermittierend jeweils das Vliesband 30 hinter dem rückwärtigen, nicht versiegelten Ende 15a des Streifenabschnitts 15 auf dem davor befindlichen Vliesbandabschnitt 11 fest, um, wie nachstehend beschrieben, den Vliesbandabschnitt 11 vom Vliesband 30 trennen zu können.

Den Klemmbacken 81 ist eine Rückholband-Anbring- und Knotvorrichtung 90 nachgeschaltet, die gemäß der DE-PS 1 491 160 ausgebildet sein kann und daher nicht näher dargestellt ist. Es genügt der Hinweis, daß das Rückholband 13 mittels dieser Vorrichtung um den Vliesbandabschnitt 11 herumgelegt und seine freien Enden miteinander verknotet werden, wenn eine Trenn- und Transportzange 110, wie nachstehend beschrieben, den Viesbandabschnitt 11 vorgezogen hat. Das Rückholband 13 hängt von dem das Rückholende 10c des Tampons 10 bildenden Längsrand 11b des Vliesbandabschnitts 11 herab (nicht dargestellt). Der Vliesbandabschnitt 11 ist dabei durch eine Öffnung 83 der Vorrichtung 90 hindurchgeführt.

Anschließend ist eine Wickelstation 100 vorgesehen, die zur Herstellung des Tamponrohlings 12 dient und der die Trenn- und Transportzange 110 nachgeschaltet ist, die in Höhe der Bewegungsbahn des Vliesbandes 30 in und entgegen dessen Bewegungsrichtung bei zurückgezogenem Wickeldorn 33 durch die Öffnung 83 der Vorrichtung 90 hindurch hin und her bewegbar und in der DE-PS 915 382 beschrieben ist. Diese Trenn- und Transportzange 110 erfaßt das vordere freie Ende 11d des Vliesbandes 30 und zieht dieses unter Durchtrennung an der Schwachstelle 31 hinter den das Vliesband 30 festklemmenden Klemmbacken 81 in den Wirkungsbereich der Rückholband-Anbring- und -Knotvorrichtung 90 sowie der Wickelstation 100, in welchen der gebildete Vliesbandabschnitt 11 in gestreckter Lage gehalten wird. In diesem Augenblick wird der gabelförmige Wickeldorn 33 aus seiner aus der Bewegungsbahn des Vliesbandes 30 zurückgezogenen Stellung axial mit seinen Gabelzinken über bzw. unter den Vliesbandabschnitt 11 vorwärts bewegt, so daß der Vliesbandabschnitt 11 sich in dem Gabelspalt befindet und der Wickeldorn noch geringfügig über die Breite des Vliesbandabschnittes 11 hinaus vorsteht. Der Wickeldorn 33 übergreift den Vliesbandabschnitt 11 in einem Querschnittsbereich, der in Bewegungsrichtung x hinter der Längsmitte des Vliesbandabschnitts 11 liegt, so daß das rückwärtige Ende 11a des Vliesbandabschnitts 11 (Fig. 3) dessen vorderes Ende 11d beim Aufwickeln in Umfangsrichtung des Tamponrohlings 12 überlappt.

Parallel zur Wickelachse sind vorzugsweise durch elektrische Widerstandsheizung beheizbare Streichbacken 101 an diametral gegenüberliegenden Seiten des Wickeldorns 33 angeordnet, die radial zur Wickelachse gegen den Tamponrohling 12 mittels einer nicht dargestellten Steuervorrichtung andrückbar sind. Die Streichbacken 101 werden auf eine Temperatur erhitzt, die ausreicht, um die aufeinanderliegenden Schmelzfasern des frei überstehenden, unversiegelten Endes 15a des Streifenabschnitts 15 mit sich selbst zu verschmelzen und dadurch eine in Umfangsrichtung feste, geschlossene Hülle aus nicht gewebtem Material zu schaffen. Die Temperatur und die Andruckkraft der Streichbacken 101 können somit geringer bemessen sein als die Temperatur und die Andruckkräfte der Siegelrolle 62.

Der Weitertransport des Tamponrohlings 12 zu einer Tamponpresse kann mit Hilfe einer Einrichtung durchgeführt werden, wie sie in der DE-PS 1 938 942 beschrieben ist. Die Presse kann entsprechend der DE-PS 1 491 161 ausgebildet sein. Die rundkuppenartige Verjüngung 10b am Einführende 10a des Tampons 10 wird nach dem Pressen mittels eines beheizten Formstempels hergestellt, wie er in der DE-PS 1 491 200 beschrieben ist.

Fig. 8 zeigt eine schematische Darstellung einer abgeänderten Ausführungsform der Einrichtung zum Herstellen des Tampons in Fig. 6, wobei gegenüber der Ausführungsform in Fig. 7 unveränderte Teile die gleichen Bezugszeichen tragen.

Eine Druckrolle 200 ist unmittelbar der Schwächungsstation 51 nachgeschaltet. Im Gegensatz zur Ausführungsform in Fig. 7 fehlt in Fig. 8 die Anordnung des Siegelbandes 65. Für die Siegelung ist hier allein eine Siegelrolle 201 vorgesehen, die weiter unten näher beschrieben ist.

In Höhe der Siegelrolle 201 befindet sich unterhalb der Bewegungsbahn des Vliesbandes 30 ein Riemenförderer 202, der aus einer Vielzahl von vorderen Antriebsscheiben 203 und rückwärtigen Umlenkscheiben 204 besteht, die jeweils endlose Führungsriemen 205 tragen.

Die Führungsriemen 205 werden im Obertrum von einer Stützrolle 206 abgestützt, der auf der Oberseite der Bewegungsbahn des Vliesbandes 30 eine Druckrolle 207 wesentlich größeren Durchmessers gegenüberliegt. Die Stützrolle 206 und die Druckrolle 207 stehen auf Klemmung gegeneinander, so daß das Vliesband 30 festgehalten wird, wenn der in Bewegungsrichtung dahinterliegende Vliesbandabschnitt 11 mittels der hinter dem Riemenförderer 202 vorgesehenen Transport- und Trennzange 110 aus dem Transportsystem herausgezogen wird. Dem Obertrum der Führungsriemen 205 ist eine Umlenkrolle 208 zugeordnet, von der aus das Obertrum der Führungsriemen 205 in Richtung schräg abwärts zu den Umlenkscheiben 204 hin abgelenkt wird.

Oberhalb der Bewegungsbahn des Vliesbandes 30 befindet sich eine Führungsvorrichtung 209, die ebenfalls aus mehreren, axial im Abstand gegeneinander versetzten Umlenkscheiben 210, 211 besteht, auf denen wiederum axial versetzt endlose Riemen 212 angeordnet sind, die jeweils oberhalb der Führungsriemen 205 des unteren Riemenförderers 202 angeordnet sind. Die vorderen Umlenkscheiben 210 der Führungsvorrichtung 209 befinden sich dicht hinter der Druckrolle 207, während die rückwärtigen Umlenkscheiben 211 oberhalb der rückwärtigen Umlenkscheiben 204 des Riemenförderers 202 angeordnet sind.

Dem Untertrum der endlosen Riemen 212 der Führungsvorrichtung 209 ist in der Nähe der Umlenkscheiben 211 eine Umlenkrolle 227 zugeordnet, von der das Untertrum schräg aufwärts zu den Umlenkscheiben 211 gerichtet ist. Infolgedessen bilden das Obertrum der Führungsriemen 205 und das Untertrum der endlosen Riemen 212 in Bewegungsrichtung des Vliesbandes 30 hinter den Umlenkrollen 208 und 227 einen sich öffnenden Spalt 228, in den die Trenn- und Transportzange 110 zum taktweisen Erfassen des jeweils vorderen Endes des Vliesbandes 30 durch die Öffnung 83 der Rückholband-Anbring- und Knotvorrichtung 90 hindurch hineinbewegbar ist.

Die rückwärtigen Umlenkscheiben 211 und 204 der oberen Führungsvorrichtung 209 bzw. des unteren Riemenförderers 202 sind auf jeweils 180° mit radial nach außen vorstehenden Führungssegmenten 213 und 214 versehen, die synchron zueinander laufen und das Vliesband 30 tragen bzw. führen, wenn die Transport- und Trennzange 110 sich entgegen der Transportrichtung x des Vliesbandes 30 in Richtung des Spaltes 228 zwischen den Umlenkscheiben 211 und 204 bewegt, um das vordere Ende des Vliesbandes 30 zu erfassen und dieses mit einer gegenüber der Transportgeschwindigkeit des Vliesbandes erhöhten Geschwindigkeit in den Wirkungsbereich der Rückholband-Anbring- und Knotvorrichtung 90 und der Wickelstation 100 zu ziehen. Da sich die Druckrolle 207 mit der Stützrolle 206 in einem Abstand von dem Spalt 228 zum Erfassen des vorderen Endes des Vliesbandes 30 durch die Transport- und Trennzange 110 befindet, der größer als die Länge eines Vliesbandabschnittes 11 bemessen ist, befindet sich die Schwachstelle 31 des Vliesbandes 30 jeweils hinter der Druckrolle 207 und der Stützrolle 206, so daß die Transport- und Trennzange 110 den sich bis zu der Schwachstelle 31 hinter der Druckrolle 207 erstreckenden Abschnitt des Vliesbandes 30 wegzieht und an der Schwachstelle 31 abreißt. Nach dieser Vereinzelung des Vliesbandabschnittes 11 wird dieser mittels der Transportzange 110 in den Bereich der Rückholband-Anbring- und -Knotvorrichtung 90 und der Wickelstation 100 geführt. Während das Anbringen des Rückholbandes 13 und das Verknoten desselben wie in Verbindung mit Fig. 7 beschrieben stattfinden kann, ist die Wickelstation 100 ebenfalls mit einem Wickeldorn 33 ausgerüstet, der axial hin und her bewegbar sowie im Drehsinn antreibbar ist. Jedoch sind in der Wickelstation 100 gemäß Fig. 8 die Streichbacken 215, 216 zum Versiegeln des freien überstehenden Endes 15a des Streifenabschnitts 15 mit dem auf dem Tamponrohling 12 befestigten Teil des Streifenabschnitts 15 unterschiedlich ausgebildet.

Die untere und obere Streichbacke 215, 216 sind im radialen Abstand vom Wickeldorn 33 zugeordnet, wobei vorzugsweise nur die untere Streichbacke 215 beheizbar ist, die sich im 3. Quadranten eines Kreises über einen Winkel von etwa 190 bis 280° erstreckt. Durch eine Durchbrechung 217 der unteren Streichbacke 215 hindurch ist ein beheizbares Siegelelement 218 gegenüber dem Wickeldorn 33 hin und her bewegbar angeordnet, das zum Ansiegeln des bislang unversiegelten rückwärtigen Endes 15a des Streifenabschnitts 15 auf einen Teil des auf dem Umfang des Tamponrohlings 12 befestigten Streifenabschnitts 15 vorgesehen ist. Da die Strinfläche 218a des Siegelelementes 218 schmal ist, ist eine nur mantellinienförmige Verschweißung des äußeren Randes des freien Endes 15a des Streifenabschnitts 15 mit dem auf dem Umfang des Tamponrohlings 12 befestigten Material desselben Streifenabschnitts 15 möglich.

Fig. 9 zeigt eine Draufsicht auf die Trenn- und Transportzange 110, deren Zangenmaul aus drei Zinkenpaaren 110a besteht, welche in der gezeigten Greifstellung in den Zinken entsprechende Zwischenräume zwischen den Führungssegmenten 213 bzw. 214 der Führungsvorrichtung 209 bzw. des Riemenförderers 202 eingreifen. Das Untertrum der endlosen Riemen 212 und das Obertrum der Führungsriemen 205 sind in einem Abstand voneinander angeordnet, durch den das Vliesband 30 nicht zusammengepresst, sondern lediglich ohne wesentliche Preßfunktion mitgeführt wird, so daß das Herausziehen des Vliesbandabschnitts 11 zwischen den erwähnten Trumen mittels der Trenn- und Transportzange 110 einschließlich des Abreißens von dem nachfolgenden Vliesband 30 an der Schwachstelle 31 desselben ohne weiteres gewährleistet ist.

Für das Zuführen, Zuschneiden und Überführen des thermoplastischen Streifenabschnitts 15 zur Vakuumrolle 71 sind bei der Ausführungsform gemäß Fig. 8 dieselben Vorrichtungen wie in Fig. 7 vorgesehen. In Fig. 8 ist lediglich der Aufbau der Vakuumrolle 71 insofern näher ersichtlich, als im Inneren der Vakuumrolle 71 ein Schieber 219 angeordnet ist, der feststeht und über seinen Umfang von etwa 180° hinweg mit seitlichen Saugöffnungen 220 versehen ist, die eine Saugwirkung über sich daran anschließende kleinere Saugöffnungen 221 in der Umfangswandung der Vakuumrolle 71 ermöglichen.

Fig. 8 zeigt in vergrößerter Darstellung die besondere Ausbildung der Siegelrolle 201, die es ermöglicht, ohne ein endloses Siegelband die Versiegelung lediglich auf einem Teilumfang der Siegelrolle 201 durch beheizbare Siegelelemente 223, 224 vorzunehmen, die auf einem Grundkörper 222 diametral gegenüberliegend angeordnet und entsprechend dem Schweiß- oder Siegelmuster profiliert sind. Die Umfangslänge der Siegelelemente 223 bzw. 224 entspricht jeweils genau der Länge eines Streifenabschnitts 15, der auf den Vliesbandabschnitt 11 aufgesiegelt werden soll. Zwei nicht beheizte Isolierelemente 225, 226 sind auf dem Grundkörper 222 befestigt, die um 90° gegenüber den diametral gegenüberliegenden Siegelelementen 223, 224 versetzt sind. Die Umfangsbögen der Isolierelemente 225, 226 haben den gleichen Radius wie die Außenflächen der Siegelelemente 223, 224, so daß sich eine Umfangsfläche aus Kreissektoren gleichen Radius' ergibt. Durch die Anordnung der Isolierelemente 225, 226 wird sichergestellt, daß jeweils das entgegen der Drehrichtung der Siegelrolle 201 über die Siegelelemente 223 bzw. 224 überstehende freie bzw. unversiegelte Ende 15a der thermoplastischen Streifenabschnitte 15 auf eines der Isolierelemente 225, 226 zu liegen kommt und infolgedessen nicht gegen das Vliesband 30 gesiegelt wird.

Um zu gewährleisten, daß die Streifenabschnitte 15 aus dem thermoplastischen, nicht gewebten Material jeweils genau auf die Siegelelemente 223 bzw. 224 zu liegen kommen, werden, wie weiter oben bereits erwähnt wurde, die Schneidwalzen 69 etwa mit der halben Umfangsgeschwindigkeit der Vakuumrolle 71 gedreht. Die Schneidwalzen 69 durchschneiden den Streifen 32 im wesentlichen, aber nicht vollständig, so daß beiderseits dieses Schnitts der nicht gewebte Materialstreifen 32 weiterhin über nicht gezeigte Materialstege ähnlich wie bei einer Perforierung verbunden bleibt. Die nachgeordnete Beschleunigungswalze 72 wird mit einer Umdrehungsgeschwindigkeit angetrieben, die der Umfangsgeschwindigkeit der Vakuumrolle 71 und damit der Transportgeschwindigkeit des Vliesbandes 30 entspricht. Kommt das vordere Ende des nicht gewebten Materialstreifens 32 in den Wirkungsbereich der Beschleunigungswalze 72, wird der vor der Schnittstelle liegende Abschnitt 15 des Materialstreifens 32 abgerissen und auf die Vliesbandgeschwindigkeit derart beschleunigt, daß die einzelnen Streifenabschnitte 15 im jeweils richtigen Abstand voneinander an das in Drehrichtung vordere Ende eines der beiden Siegelelemente 223, 224 herangeführt werden.

Der hinter der Abreißstelle befindliche Materialstreifen 32 wird infolge der Saugwirkung der Vakuumrolle 71 stets in Transportrichtung geglättet und gestreckt. Aufgrund der außerordentlich geringen Masse des abgetrennten Streifenabschnitts 15 wird dieser schlagartig auf die Vliesbandgeschwindigkeit beschleunigt, so daß eine stets genaue Positionierung des Streifenabschnitts 15 auf der Vakuumrolle 71 erreicht werden kann.

Wie aus Fig. 8 ersichtlich ist, endet ein Schlitz 219a des Schiebers 219 der Vakuumrolle 71 kurz vor dem Walzenspalt, den die Vakuumrolle 71 mit der Siegelrolle 201 bildet. Im Bereich des Spaltes zwischen Vakuumrolle 71 und Siegelrolle 201 wird der thermoplastische Streifenabschnitt 15 so stark erwärmt, daß er plastisch und klebrig wird und infolgedessen an der Siegelrolle 201 anhaftet und von dieser schlupffrei mitgenommen wird, aber die sichere Über- und Mitnahme des Streifenabschnitts 15 durch den Vliesabschnitt 11 nach dem Versiegeln gewährleistet ist.

Aus der vorstehenden Beschreibung ist somit ersichtlich, daß die Einrichtung gemäß Fig. 8 einen kontinuierlichen Transport des Vliesbandes 30 bis zur Rückholband- Anbring- und Knotvorrichtung 90 bzw. der Wickelstation 100 ermöglicht, wobei von den das Vliesband 30 transportierenden Einrichtungen lediglich die Trenn- und Transportzange 110 eine hin und hergehende Bewegung ausführt, während alle anderen Vorrichtungen ständig in einer Richtung umlaufen. Es ist ferner ersichtlich, daß mit den beschriebenen Einrichtungen nicht nur eine hohe Produktionsgeschwindigkeit erreicht wird, wie sie für die Massenproduktion erforderlich ist, sondern daß hierdurch auch die wirtschaftliche Massenproduktion eines wesentlich verbesserten Tampons für die Frauenhygiene bei gleichbleibend hoher Qualität erreicht werden kann.

## Patentansprüche

1. Tampon für die Frauenhygiene, bestehend aus einem Abschnitt (11) bestimmter Länge eines sich aus wirr liegenden Natur- und/oder Kunststoffasern zusammensetzenden Vliesbandes (30) mit einer der Länge des Tampons (10) etwa entsprechenden Breite und einem nicht gewebten, thermoplastischen, an einem Ende des Vliesbandabschnitts (11) durch Heißsiegeln befestigten Streifenabschnitt (15) aus flüssigkeitsdurchlässigem, mindestens teilweise thermoplastischen Material, wobei der Vliesbandabschnitt (11) im wesentlichen auf sich selbst zu einem mit einem Rückholband (13) versehenen Tamponrohling (12) aufgewickelt ist, auf dessen Umfang der Streifenabschnitt (15) gerollt ist und der auf die Endform des Tampons (10) im wesentlichen radial gepreßt ist, dadurch gekennzeichnet, daß der flüssigkeitsdurchlässige, thermoplastische Streifenabschnitt (15) auf einer Länge mit der Außenseite des Vliesbandabschnitts (11) durch Heißsiegeln verbunden ist, die etwa der Länge des Umfangs des Tamponrohlings (12) entspricht, und daß das äußere, über das Ende des Vliesbandabschnitts (11) überstehende Ende (15a) des Streifenabschnitts (15) mit der Außenseite eines Teils des auf den Vliesbandabschnitt (11) aufgesiegelten, aus Bikomponentenfasern bestehenden Streifenabschnitts (15) verschweißt ist.

2. Tampon nach Anspruch 1, dadurch gekennzeichnet, daß die Schweißverbindung zwischen dem Streifenabschnitt (15) und dem Vliesbandabschnitt (11) auf im Abstand voneinander angeordneten Stellen vorgesehen ist.

3. Tampon nach Anspruch 2, dadurch gekennzeichnet, daß die Schweißstellen zwischen dem Streifenabschnitt (15) und dem Vliesbandabschnitt (11) punkt- und/oder linienförmig ausgebildet sind.

4. Tampon nach Anspruch 1, dadurch gekennzeichnet, daß der thermoplastische, flüssigkeitsdurchlässige Streifenabschnitt (15) schmaler als die Breite des Vliesbandabschnitts (11) ist, aber den das Rückholende (10c) des Tampons (10) bildenden Längsrand (11b) desselben überdeckt.

5. Tampon nach Anspruch 4, dadurch gekennzeichnet, daß der das Einführende (10a) bildende Längsrand (11c) des Vliesbandabschnitts (11) über den zugehörigen Rand des flüssigkeitsdurchlässigen, thermoplastischen Streifenabschnitts (15) auf einer Breite vorsteht, die etwa der Höhe einer Verjüngung (10b) des Tampons (10) an dessen Einführende (10a) entspricht.

6. Tampon nach Anspruch 1, dadurch gekennzeichnet, daß die Bikomponentenfaser aus einem Polyester-Kern und einer Ummantelung aus Hochdruck-Polyäthylen besteht, das einen gegenüber dem Polyester niedrigeren Schmelzpunkt aufweist.

7. Verfahren zum Herstellen des Tampons nach einem der Ansprüche 1 bis 6, bei dem ein Längenabschnitt eines flüssigkeitsdurchlässigen, thermoplastischen, ungewebten Materialstreifens (32) auf die Außenseite des rückwärtigen Endes eines Vliesbandabschnitts (11) bestimmter Länge aufgeschweißt wird, dessen Breite der Länge des Tampons (10) etwa entspricht, woraufhin der Vliesbandabschnitt (11) mit einem Rückholband (13) versehen und auf sich selbst zur Bildung eines Tamponrohlings (12) aufgerollt wird, so daß der Streifenabschnitt (15) sich über den Umfang des Tamponrohlings (12) erstreckt, welcher anschließend auf die Endform des Tampons (10) gepreßt wird, dadurch gekennzeichnet, daß der nicht gewebte thermoplastische, sich aus Bikomponenten zusammensetzende Streifenabschnitt (15) auf einer der Umfangslänge des Tamponrohlings (12) etwa entsprechenden Länge auf den Vliesbandabschnitt (11) aufgesiegelt wird und nach dem Aufrollen des Vliesbandabschnitts (11) zu dem Tamponrohling (12) das über den Vliesbandabschnitt (11) hinausragende unversiegelte Ende (15a) mit dem Streifenabschnitt (15) unter Anwendung von Hitze und Druck verschweißt wird.

8. Verfahren nach Anspruch 7, dadurch gekennzeichnet, daß das Vliesband (30) kontinuierlich zugeführt und auf für die Herstellung des Tampons (10) notwendigen Längenabschnitten quer zu seiner Längsrichtung geschwächt und an der Schwachstelle (31) unter Bildung eines Vliesbandabschnitts (11) durchtrennt wird, welcher mit einem Rückholband (13) versehen und gleichzeitig in Bewegungsrichtung des Vliesbandes (30) vor dessen Längsmitte beginnend im wesentlichen auf sich selbst zu einem Tamponrohling (12) aufgerollt wird, so daß sich die Enden des Vliesbandabschnitts (11) in Umfangsrichtung überlappen.

9. Verfahren nach Anspruch 8, dadurch gekennzeichnet, daß beim Aufsiegeln des thermoplastischen Streifenabschnitts (15) auf das Vliesband (30) Hitze und Druck stärker angewandt werden als beim Verschweißen des über das äußere Ende (11a) des Vliesbandabschnitts (11) sich frei hinaus erstreckenden unversiegelten Endes (15a) des Streifenabschnitts (15) mit dem auf den Vliesbandabschnitt (11) aufgesiegelten Streifenabschnitt (15).

10. Verfahren nach den Ansprüchen 8 oder 9, dadurch gekennzeichnet, daß die Länge des sich über das äußere Ende (11a) des aufgewickelten Vliesbandabschnitts (11) hinaus erstreckenden unversiegelten Endes (15a) des Streifenabschnitts (15) zwischen 10 und 50 mm beträgt.

11. Verfahren nach einem der Ansprüche 8 bis 10, gekennzeichnet durch die Verwendung eines flüssigkeitsdurchlässigen, thermoplastischen Streifen (32), der schmaler als das Vliesband (30) ist und der sich von dem das Rückholende (10c) des Tampons (10) bildenden Längsrand (11b) des Vliesbandes (30) nur bis in die Nähe des das Einführende (10a) des Tampons (10) bildenden Längsrandes (11c) des Vliesbandes (30) erstreckt.

12. Verfahren nach Anspruch 11, dadurch gekennzeichnet, daß das vom thermoplastischen Streifenabschnitt (15) unbedeckte Einführende (10a) des Tampons (10) nach dem radialen Pressen des Tamponrohlings (12) kugelkalottenartig verformt wird.

13. Verfahren nach Anspruch 7, dadurch gekennzeichnet, daß eine Bikomponentenfaser mit einem Polyester-Kern und einer HD-Polyäthylen-Ummantelung verwendet wird.

14. Einrichtung zur Herstellung des Tampons nach einem der Ansprüche 1 bis 6 unter Ausführung des Verfahrens nach einem der Ansprüche 7 bis 13, bestehend aus folgenden, in Verarbeitungsrichtung hintereinander geschalteten Vorrichtungen :
einer Vorratsrolle (50) für ein fortlaufendes Vliesband (30) aus Natur- und/oder Kunststofffasern ;
einer Schwächungsstation (51) für das Vliesband (30) ;
einer Transportvorrichtung (55, 56) für das Vliesband (30) ;
einer Vliesband-Trennvorrichtung (80) zum fortlaufenden Abtrennen von Vliesbandabschnitten (11) ;
einer Rückholband-Anbring- und -Knotvorrichtung (90) ;
einer Wickelstation (100) zum Aufrollen des Vliesbandabschnitts (11) unter Bildung eines Tamponrohlings (12),
einer etwa in der Transportebene des Vliesbandes (30) hin- und herbewegbaren Transport- und Trennzange (110) zum Abtrennen je eines Vliesbandabschnitts (11), und einer Vorrichtung zum Überführen des Tamponrohlings (12) zu einer Presse, in welcher der Tamponrohling (12) auf die Endform des Tampons (10) im wesentlichen radial preßbar ist, sowie einer Formvorrichtung für das Einführende (10a) des Tampons, dadurch gekennzeichnet, daß eine Vorratsrolle (66) für einen fortlaufenden Streifen (32) aus nicht gewebtem, flüssigkeitsdurchlässigen, thermoplastischen Material von einer gegenüber dem Vliesband (30) geringeren Breite vorgesehen ist, welcher eine Schneidstation (70) zum überwiegenden Durchtrennen des fortlaufenden Streifens (32) in Abschnitte (15) bestimmter Länge nachgeschaltet ist, hinter der eine Abtrenn- und Übergabevorrichtung (82) für die Streifenabschnitte (15) angeordnet ist, der eine Siegelstation (60) zum Aufsiegeln des thermoplastischen Streifenabschnitts (15) auf die Außenseite des Vliesbandes (30) nachgeschaltet ist, welcher eine Vorrichtung für den kontinuierlichen Transport des Vliesbandes (30) mit dem darauf aufgesiegelten Streifenabschnitt (15) zu der Vliesband-Trennvorrichtung (80) nachgeordnet ist, und daß der Wickelstation (100) eine beheizbare Schweiß- und Glättvorrichtung zum Andrücken des sich über das rückwärtige Ende (11a) des Vliesbandabschnitts (11) frei und unversiegelt erstreckenden Endes (15a) des Streifenabschnitts (15) gegen den auf den Vliesbandabschnitts (15) aufgesiegelten Streifenabschnitt (15) zugeordnet ist.

15. Einrichtung nach Anspruch 14, dadurch gekennzeichnet, daß die Schneidstation (70) für den flüssigkeitsdurchlässigen thermoplastischen Streifen (32) aus einem Paar Schneidwalzen (69, 69a) besteht, von denen die Messerwalze (69) auf einem wesentlichen Teil einer Mantellinie mit Schneiden für die überwiegende Durchtrennung des Streifens (32) versehen ist, wobei die Schneidwalzen (69, 69a), jeweils oberhalb und unterhalb der Bewegungsbahn (x) des Vliesbandes (30) angeordnet und mit einer Umfangsgeschwindigkeit antreibbar sind, die etwa der halben Transportgeschwindigkeit des Vliesbandes (30) entspricht.

16. Einrichtung nach Anspruch 14, dadurch gekennzeichnet, daß die Abtrenn- und Übergabevorrichtung (82) für den flüssigkeitsdurchlässigen, thermoplastischen Streifenabschnitt (15) eine Vakuumrolle (71) ist, in deren hohlem Innenraum ein Schieber (219) vorgesehen ist, der an eine Vakuumquelle anschließbar ist und dessen Saugöffnungen (220) mit Saugöffnungen (221) der Vakuumrolle (71) verbindbar sind, wobei eine Abtrenn- und Beschleunigungswalze (72) für den Streifenabschnitt (15) am Umfang der Vakuumrolle (71) in einem Abstand von der Schneidstation (70) angeordnet ist, der größer als die Länge eines Streifenabschnitts (15) bemessen ist, wobei sowohl die Vakuumrolle (71) als auch die Beschleunigungswalze (72) mit einer Umfangsgeschwindigkeit antreibbar sind, die der Transportgeschwindigkeit des Vliesbandes (30) entspricht.

17. Einrichtung nach Anspruch 14, dadurch gekennzeichnet, daß die Siegelstation (60) sich aus einer Druckrolle (57) an der Unterseite der Bewegungsbahn des Vliesbandes (30) und an der Oberseite derselben aus einer Siegelrolle (62 ; 201) zusammensetzt, die beheizbar ist.

18. Einrichtung nach Anspruch 15, dadurch gekennzeichnet, daß die Siegelrolle (62) eine Umlenkrolle für ein endloses Siegelband (65) bildet, dessen Material gegenüber der Siegeltemperatur hitzebeständig ist und das um weitere Umlenkrollen (63, 64) geführt ist.

19. Einrichtung nach Anspruch 17, dadurch gekennzeichnet, daß die Druckrolle (57) zum Aufschweißen des thermoplastischen Streifenabschnitts (15) zwischen einer Ruhestellung und einer Anpreßstellung mittels einer Steuervorrichtung gegen die Siegelrolle (62) intermittierend bewegbar ist.

20. Einrichtung nach Anspruch 17, dadurch gekennzeichnet, daß der Siegelrolle (62 ; 201) mindestens ein endloses Transportelement (73 ; 205) unterhalb der Bewegungsbahn des Vliesbandes (30) zugeordnet ist.

21. Einrichtung nach Anspruch 20, dadurch gekennzeichnet, daß eine Umlenkrolle (75) für ein endloses Transportband (73) in einen Zwischenraum zwischen den beiden gegenüberliegenden Umlenkrollen (63, 64) für das Siegelband (65) eingreift.

22. Einrichtung nach einem der Ansprüche 17 bis 21, dadurch gekennzeichnet, daß zwei beheizbare Streichbacken (101) im wesentlichen radial zur Achse des Wickeldornes (33) oberhalb und unterhalb desselben mittels einer Steuervorrichtung hin und her bewegbar sind, so daß das freie Ende (15a) eines thermoplastischen Streifenabschnitts (15) auf dem Tamponrohling (12) mit dem letztgenannten verschweißt werden kann.

23. Einrichtung nach Anspruch 17, dadurch gekennzeichnet, daß der Umfang der Siegelrolle (201) mit beheizbaren Siegelelementen (223, 224) sowie nicht beheizbaren Isolierelementen (225, 226) bestückt ist, die in Umfangsrichtung der Siegelrolle (201) alternierend angeordnet sind.

24. Einrichtung nach Anspruch 23, dadurch gekennzeichnet, daß die Bogenlänge der Siegelelemente (223, 224) der Länge des auf das Vliesband (30) aufzusiegelnden Abschnitts des Streifenabschnitts (15) etwa entspricht.

25. Einrichtung nach den Ansprüchen 17 und 20, dadurch gekennzeichnet, daß der Siegelrolle (201) oberhalb der Bewegungsbahn des Vliesbandes (30) eine Druckrolle (207) nachgeschaltet ist, welcher unter dem Obertrum eines Riemenförderers (202), der unter der Bewegungsbahn des Vliesbandes (30) angeordnet ist, eine Stützrolle (206) zugeordnet ist.

26. Einrichtung nach Anspruch 25, dadurch gekennzeichnet, daß hinter der Druckrolle (207) oberhalb der Bewegungsbahn des Vliesbandes (30) eine Führungsvorrichtung (209) für das Vliesband (30) angeordnet ist.

27. Einrichtung nach den Ansprüchen 25 und 26, dadurch gekennzeichnet, daß die Führungsvorrichtung (209) aus mehreren endlosen Riemen (212) besteht, die um Umlenkscheiben (210, 211) in derselben Ebene herumgeführt sind, in der die endlosen Transportelemente (205) des Riemenförderers (202) gelagert sind, wobei die Transporttrume der Führungsvorrichtung (209) und des Riemenförderers (202) an der Ausgangsseite mittels Umlenkrollen (208, 227) einen sich erweiternden Spalt (228) für den Eingriff der Trenn- und Transportzange (110) zum Erfassen des vorderen Endes des Vliesbandes (30) bilden.

28. Einrichtung nach den Ansprüchen 25 bis 27, dadurch gekennzeichnet, daß die vor der Rückholband-Anbring- und -knotstation (90) befindlichen Umlenkscheiben (211, 204) der Führungsvorrichtung (209) und des Riemenförderers (202) jeweils mit radial nach außen vorstehenden Führungssegmenten (213, 214) versehen sind, die sich jeweils über einen Teil des Umfangs der Umlenkscheiben (211, 204) erstrecken und zur Führung des Vliesbandes (30) bzw. Vliesbandabschnitts (11) zusammenwirken.

29. Einrichtung nach Anspruch 14, dadurch gekennzeichnet, daß im radialen Abstand oberhalb und unterhalb von dem Wickeldorn (33) Streichbacken (215, 216) für das freie Ende (15a) eines Streifenabschnitts (15) vorgesehen sind, von denen die obere Streichbacke (216) symmetrisch zu einer senkrechten, durch die Achse des Wickeldorns verlaufenden Hauptebene angeordnet ist, während sich die untere Streichbacke (215) im wesentlichen im dritten Quadranten eines Kreises über einen Umfangswinkel von 180° bis 270° erstreckt.

30. Einrichtung nach Anspruch 29, dadurch gekennzeichnet, daß die untere Streichbacke (215) mit einer Durchbrechung (217) versehen ist, durch welche hindurch ein beheizbares Siegelelement (215) etwa radial zum Wickeldorn (33) mittels einer Steuervorrichtung hin- und herbewegbar angeordnet ist, so daß das freie Ende (15a) des Streifenabschnitts (15) mit diesem entlang einer Mantellinie des Tamponrohlings (12) versiegelbar ist.

31. Einrichtung nach Anspruch 14, dadurch gekennzeichnet, daß die Schwächungsstation (51) für das Vliesband (30) der Siegelstation (60) vorgeschaltet ist und aus einem Paar Perforations- und Klemmwalzen (53) sowie einem Paar Streckwalzen (54) besteht, die in Bewegungsrichtung (x) des Vliesbandes (30) hintereinander geschaltet sind.

32. Einrichtung nach einem der Ansprüche 14 und 31, dadurch gekennzeichnet, daß der Schwächungsstation (51) ein endloses Tragband (56) für das Vliesband (30) nachgeschaltet ist, wobei der in Transportrichtung (x) des Vliesbandes (30) vorderen Antriebs- und Spannrolle (56a) für das Transportband (56) eine Transportwalze (55) zugeordnet ist, die an der Oberseite des Vliesbandes (30) angreift.

33. Einrichtung nach Anspruch 14, dadurch gekennzeichnet, daß die Vliesbandtrennvorrichtung (80) aus einem Paar schwenkbarer Klemmbacken (81) besteht, die jeweils oberhalb und unterhalb der Bewegungsbahn (x) des Vliesbandes (30) vor der Rückholband-Anbring- und Knotvorrichtung (90) angeordnet sind und mit der Trenn- und Transportzange (110) zusammenwirken, um einen Vliesbandabschnitt (11) vom Vliesband (30) abzutrennen.

**Claims**

1. Tampon for female hygiene, consisting of a section (11) of specific length of a nonwoven ribbon (30) which is composed of randomly laid natural and/or synthetic fibres and has a width approximately corresponding to the length of the tampon (10), and of a nonwoven, thermoplastic strip section (15) fixed by heat-sealing to one end of the nonwoven ribbon section (11), of a liquid-permeable, at least partially thermoplastic material, the nonwoven ribbon section (11) being wound essentially upon itself to give a tampon blank (12) which is provided with a withdrawal cord (13) and upon the circumference of which the strip section (15) is rolled and which is pressed essentially radially to give the final form of the tampon (10), characterized in that the liquid-permeable, thermoplastic strip section (15) is bonded by heat-sealing to the outside of the nonwoven ribbon section (11) over a length which approximately corresponds to the length of the circumference of the tampon blank (12), and in that the outer end (15a) of the strip section (15), which projects beyond the end of the nonwoven ribbon section (11), is welded to the outside of a part of the strip section (15) which consists of bicomponent fibres and is sealed to the nonwoven ribbon section (11).

2. Tampon according to Claim 1, characterized in that the welded bond between the strip section (15) and the nonwoven ribbon section (11) is provided at points arranged at a mutual distance.

3. Tampon according to claim 2, characterized in that the welding points between the strip section (15) and the nonwoven ribbon section (11) are in the form of spots and/or lines.

4. Tampon according to Claim 1, characterized in that the thermoplastic, liquid-permeable strip section (15) is narrower than the width of the nonwoven ribbon section (11), but covers the longitudinal edge (11b) of the tampon (10) which forms the withdrawal end (10c) thereof.

5. Tampon according to claim 4, characterized in that the longitudinal edge (11c), forming the introduction end (10a), of the nonwoven ribbon section (11) projects beyond the associated edge of the liquid-permeable, thermoplastic strip section (15) over a width which approximately corresponds to the height of a constriction (10b) of the tampon (10) at the introduction end (10a) thereof.

6. Tampon according to Claim 1, characterized in that the bi-component fibre consists of a polyester core and a sheathing of high-pressure polyethylene which has a melting point lower than that of the polyester.

7. Process for producing the tampon according to any of Claims 1 to 6, in which a length section of a liquid-permeable, thermoplastic, nonwoven strip (32) of material is welded to the outside of the rear end of a nonwoven ribbon section (11) of specific length, the width of the nonwoven ribbon section approximately corresponding to the length of the tampon (10), whereupon the nonwoven ribbon section (11) is provided with a withdrawal cord (13) and rolled upon itself to form a tampon blank (12), so that the strip section (15) extends over the circumference of the tampon blank (12) which is subsequently pressed to give the final form of the tampon (10), characterized in that the nonwoven thermoplastic strip section (15) composed of bicomponents is sealed to the nonwoven ribbon section (11) a length approximately corresponding to the circumferential length of the tampon blank (12) and, after the nonwoven ribbon section (11) has been rolled up to give the tampon blank (12), the unsealed end (15a) projecting beyond the nonwoven ribbon section (11) is welded to the strip section (15) with the application of heat and pressure.

8. Process according to Claim 7, characterized in that the nonwoven ribbon (30) is continuously fed and weakened transversely to its longitudinal direction for producing the length sections necessary for the manufacture of the tampon (10) and severed at the weak point (31) with formation of a nonwoven ribbon section (11) which is provided with a withdrawal cord (13) and is simultaneously rolled up essentially upon itself in the direction of movement of the nonwoven ribbon (30), starting in front of the longitudinal centre thereof, to give a tampon blank (12), so that the ends of the nonwoven ribbon section (11) mutually overlap in the circumferential direction.

9. Process according to Claim 8, characterized in that when sealing the thermoplastic strip section (15) onto the nonwoven ribbon (30), more heat and pressure are applied than in welding the unsealed end (15a), extending freely beyond the outer end (11a) of the nonwoven ribbon section (11), of the strip section (15) to the strip section (15) sealed onto the nonwoven ribbon section (11).

10. Process according to Claims 8 or 9, characterized in that the length of the unsealed end (15a), extending beyond the outer end (11a) of the wound-up nonwoven ribbon section (11), of the strip section (15) is between 10 and 50 mm.

11. Process according to any of Claims 8 to 10, characterized by the use of a liquid-permeable, thermo-plastic strip (32) which is narrower than the nonwoven ribbon (30) and which extends from the longitudinal edge (11b), forming the withdrawal end (10c) of the tampon (10), of the nonwoven ribbon (30) only as far as the vicinity of the longitudinal edge (11c), forming the introduction end (10a) of the tampon (10), of the nonwoven ribbon (30).

12. Process according to Claim 11, characterized in that introduction end (10a), not covered by the thermoplastic strip section (15), of the tampon (10) is deformed in the shape of a spherical cap after the radial pressing of the tampon blank (12).

13. Process according to Claim 7, characterized in that a bi-component fibre having a polyester core and a high-pressure polyethylene sheathing is used.

14. Apparatus for producing the tampon according to any of Claims 1 to 6, carrying out the process according to any of Claims 7 to 13, consisting of the following devices arranged in series in the direction of processing :

a stock roll (50) for a continuous nonwoven ribbon (30) of natural and/or synthetic fibres ;

a weakening station (51) for the nonwoven ribbon (30) ;

a transport device (55, 56) for the nonwoven ribbon (30) ;

a nonwoven ribbon-severing device (80) for the continuous severing of nonwoven ribbon sections (11) ;

a withdrawal cord-attaching and -knotting device (90) ;

a winding station (100) for rolling up the nonwoven ribbon section (11) to form a tampon blank (12), transport and severing tongs (110), which can be moved to and fro approximately in the transport plane of the nonwoven ribbon (30), for severing one nonwoven ribbon section (11) each time, and a device for transferring the tampon blank (12) to a press in which the tampon blank (12) can be pressed essentially radially to give the final form of the tampon (10), and a device for forming the introduction end (10a) of the tampon, characterized in that a stock roll (66) for a continuous strip (32) of nonwoven, liquid-permeable, thermoplastic material of width smaller than that of the nonwoven ribbon (30) is provided, which roll is followed by a cutting station (70) for largely severing the continuous strip (32) into sections (15) of specific length, after which a separation and transfer device (82) for the strip sections (15) is arranged, which is followed by a sealing station (60) for sealing the thermoplastic strip section (15) onto the outside of the nonwoven ribbon (30), downstream of which a device for continuously transporting the nonwoven ribbon (30) with the strip section (15) sealed thereto to the nonwoven ribbon-severing device (80) is provided, and in that the winding station (100) is associated with a heatable welding and smoothing device for pressing the end (15a), extending freely and unsealed beyond the rear end (11a) of the nonwoven ribbon section (11), of the strip section (15) against the strip section (15) sealed onto the nonwoven ribbon section (11).

15. Apparatus according to Claim 14, characterized in that the cutting station (70) for the liquid-permeable, thermo-plastic strip (32) consists of a pair of cutting rollers (69, 69a), of which the blade roller (69) is provided on a substantial part of a circumferential line with cutting edges for largely severing the strip (32), the cutting rollers (69, 69a) being arranged respectively above and below the movement track (x) of the nonwoven ribbon (30) and being drivable at a circumferential speed which corresponds approximately to half the transport speed of the nonwoven ribbon (30).

16. Apparatus according to Claim 14, chacterized in that the separation and transfer device (82) for the liquid-permeable, thermoplastic strip section (15) is a vacuum roller (71), in the hollow interior of which a slide (219) is provided which can be connected to a vacuum source and the suction orifices (220) of which can be connected to suction orifices (221) of the vacuum roller (71), a separation and acceleration roller (72) for the strip section (15) being arranged on the circumference of the vacuum roller (71) at a distance from the cutting station (70), which is greater than the length of a strip section (15), both the vacuum roller (71) and the acceleration roller (72) being drivable at a circumferential speed which corresponds to the transport speed of the nonwoven ribbon (30).

17. Apparatus according to Claim 14, characterized in that the sealing station (60) is composed of a pressure roller (57) on the underside of the movement track of the nonwoven ribbon (30) and, on the upper side thereof, of a sealing roller (62 ; 201) which can be heated.

18. Apparatus according to Claim 15, characterized in that the sealing roller (62) forms a deflection roller for an endless sealing tape (65), the material of which is heat-resistant at the sealing temperature and which is taken around further deflection rollers (63, 64).

19. Apparatus according to Claim 17, characterized in that the pressure roller (57) for the welding-on of the thermoplastic strip section (15) is intermittently movable between a rest position and a contact pressure position towards the sealing roller (62) by means of a control device.

20. Apparatus according to Claim 17, characterized in that the sealing roller (62 ; 201) is associated with at least one endless transport element (73 ; 205) below the movement track of the nonwoven ribbon (30).

21. Apparatus according to Claim 20, characterized in that a deflection roller (75) for an endless transport belt (73) engages in an interspace between the two mutually opposite deflection rollers (63, 64) for the sealing tape (65).

22. Apparatus according to any of Claims 17 to 21, characterized in that two heatable spreading jaws (101) are movable to and fro essentially radially to the axis of the winding mandrel (33) above and below the latter by means of a control device, so that the free end (15a) of a thermoplastic strip section (15) can be welded to the tampon blank (12) on top of the latter.

23. Apparatus according to Claim 17, characterized in that the circumference of the sealing roller (201) is fitted with heatable sealing elements (223, 224) and with nonheatable insulating elements (225, 226), which are arranged alternately in the circumferential direction of the sealing roller (201).

24. Apparatus according to Claim 23, characterized in that the arc length of the sealing elements (223, 224) approximately corresponds to the length of the portion of the strip section (15) to be sealed to the nonwoven ribbon (30).

25. Apparatus according to Claims 17 and 20, characterized in that, downstream of the sealing roller (201) and above the movement track of the nonwoven ribbon (30), a pressure roller (207) is provided which is associated with a support roller (206) underneath the upper section of a belt conveyor (202) which is arranged below the movement track of the nonwoven ribbon (30).

26. Apparatus according to Claim 25, characterized in that guide device (209) for the nonwoven ribbon (30) is arranged after the pressure roller (207) and above the movement track of the nonwoven ribbon (30).

27. Apparatus according to Claims 25 and 26, characterized in that the guide device (209) consists of several endless belts (212) which are taken around pulleys (210, 211) in the same plane in which the endless transport elements (205) of the belt conveyor (202) are mounted, the transport sections of the guide device (209) and of the belt conveyor (202) forming on the outlet side, by means of deflection rollers (208, 227), a widening gap (228) for the engagement of the separation and transport tongs (110) for gripping the front end of the nonwoven ribbon (30).

28. Apparatus according to Claims 25 to 27, characterized in that the pulleys (211, 204), located upstream of the withdrawal cord-attaching and -knotting station (90), of the guide device (209) and of the belt conveyor (202) are each provided with radially outward-projecting guide segments (213, 214) which each extend over a part of the circumference of the pulleys (211, 204) and interact for guiding the nonwoven ribbon (30) or nonwoven ribbon section (11).

29. Apparatus according to Claim 14, characterized in that at a radial distance above and below the winding mandrel (33), spreading jaws (215, 216) for the free end (15a) of a strip section (15) are provided, of which the upper spreading jaw (216) is arranged symmetrically to a vertical main plane running through the axis of the winding mandrel, whilst the lower spreading jaw (215) extends essentially in the third quadrant of a circle over an angle from 180° to 270° at the circumference.

30. Apparatus according to Claim 29, characterized in that the lower spreading jaw (215) is provided with a perforation (217), a heatable sealing element (218) being arranged to be movable to and fro through this perforation by means of a control device approximately radially to the winding mandrel (33), so that the free end (15a) of the strip section (15) is sealable by means of this element along a circumferential line of the tampon blank (12).

31. Apparatus according to Claim 14, characterized in that the weakening station (51) for the nonwoven ribbon (30) is arranged upstream of the sealing station (60) and consists of a pair of perforation and clamping rollers (53) and a pair of stretching rollers (54), which pairs are arranged in series in the movement direction (x) of the nonwoven ribbon (30).

32. Apparatus according to Claims 14 or 31, characterized in that an endless carrier belt (56) for the nonwoven ribbon (30) is provided downstream of the weakening station (51), the driving and tensioning roller (56a) which is in front in the transport direction (x) of the nonwoven ribbon (30) for the transport belt (56) being associated with a transport roller (55) which engages on the upper side of the nonwoven ribbon (30).

33. Apparatus according to Claim 14, characterized in that the nonwoven ribbon-severing device (80) consists of a pair of pivotable clamping jaws (81) which are respectively arranged above and below the movement track (x) of the nonwoven ribbon (30) in front of the withdrawal cord-attaching and -knotting device (90) and interact with the separation and transport tongs (110) for severing a nonwoven ribbon section (11) from the nonwoven ribbon (30).

**Revendications**

1. Tampon pour l'hygiène féminine, se composant d'un segment (11) de longueur déterminée d'une bande ouatée (30) formée de fibres naturelles et/ou synthétiques entremêlées et ayant une largeur correspondant à peu près à la longueur du tampon (10), et d'un segment de ruban non tissé (15), thermoplastique, fixé par scellement à chaud à une extrémité du segment de bande (11) et formé au moins en partie d'une matière thermoplastique et perméable aux liquides, le segment de bande (11) étant dans l'essentiel enroulé sur lui-même sous la forme d'une ébauche de tampon (12) pourvue d'un cordon de retrait (13), sur la périphérie de laquelle est enroulé le segment de ruban (15) et qui est comprimée dans l'essentiel radialement à la forme finale du tampon (10), caractérisé en ce que le segment de ruban (15) thermoplastique et perméable aux liquides est relié par scellement à chaud avec le côté extérieur du segment de bande ouatée (11) sur une longueur qui correspond à peu près à la longueur de la périphérie de l'ébauche de tampon (12) et en ce que l'extrémité extérieure (15a) du segment de ruban (15) qui dépasse de l'extrémité du segment de bande (11) est soudée sur le côté extérieur d'une partie du segment de ruban (15) scellé sur le segment de bande (11) et formé de fibres à deux composants.

2. Tampon selon la revendication 1, caractérisé en ce que la liaison soudée entre le segment de ruban (15) et le segment de bande (11) est prévue en des endroits espacés l'un de l'autre.

3. Tampon selon la revendication 2, caractérisé en ce que les zones de soudure entre le segment de ruban (15) et le segment de bande (11) sont en forme de points et/ou de lignes.

4. Tampon selon la revendication 1, caractérisé en ce que le segment de ruban (15) thermoplastique et perméable aux liquides est plus étroit que la largeur du segment de bande (11) mais recouvre cependant le bord longitudinal (11b) de ce dernier, formant l'extrémité de retrait (10c) du tampon (10).

5. Tampon selon la revendication 4, caractérisé en ce que le bord longitudinal (11c), formant l'extrémité d'introduction (10a) du segment de bande (11) fait saillie au delà du bord associé du segment de ruban (15) thermoplastique et perméable aux liquides sur une largeur qui correspond à peu près à la hauteur d'un rétrécissement (10b) du tampon (10) à son extrémité d'introduction (10a).

6. Tampon selon la revendication 1, caractérisé en ce que la fibre à deux composants est constituée d'une âme en polyester et d'une enveloppe en polyéthylène à haute pression, qui a un point de fusion plus bas que celui du polyester.

7. Procédé de fabrication du tampon selon une des revendications 1 à 6, selon lequel un segment de longueur d'un ruban de matière non tissée, thermoplastique et perméable aux liquides (32) est soudé sur le côté extérieur de l'extrémité arrière d'un segment de bande ouatée (11) d'une longueur déterminée, dont la largeur correspond à peu près à la longueur du tampon (10), et ensuite le segment de bande (11) est pourvu d'un cordon de retrait (13) et est enroulé sur lui-même pour former une ébauche de tampon (12) de telle sorte que le segment de ruban (15) s'étende sur la périphérie de l'ébauche de tampon (12), qui est ensuite comprimée à la forme finale du tampon (10), caractérisé en ce que le segment de ruban (15), thermoplastique, non tissé et constitué de deux composants, est scellé sur le segment de bande (11) sur une longueur correspondant à peu près à la longueur périphérique de l'ébauche de tampon (12) et, après l'enroulement du segment de bande (11) sous la forme de l'ébauche de tampon (12), l'extrémité (15a), non scellée et dépassant du segment de bande (11), est soudée sur le segment de ruban (15) par application de chaleur et de pression.

8. Procédé selon la revendication 7, caractérisé en ce que la bande ouatée (30) est introduite de façon continue, est affaiblie perpendiculairement à sa direction longitudinale en des endroits de sa longueur qui sont nécessaires pour la fabrication du tampon (10) et est sectionnée dans la zone d'affaiblissement (31) lors de la formation d'un segment de bande (11), qui est pourvu d'un cordon de retrait (13) et qui est enroulé simultanément, dans la direction de mouvement de la bande non tissée (30), en commençant avant son milieu longitudinal, sensiblement sur elle-même sous la forme d'une ébauche de tampon (12), de telle sorte que les extrémités du segment de bande (11) se recouvrent dans la direction périphérique.

9. Procédé selon la revendication 8, caractérisé en ce que, lors du scellement du segment de ruban thermoplastique (15) sur la bande ouatée (30), on fait intervenir plus de chaleur et de pression que lors du soudage de l'extrémité (15a) du segment de ruban (15), qui est non scellée et qui dépasse librement de l'extrémité extérieure (11a) du segment de bande (11), avec le segment de ruban (15) scellé sur le segment de bande (11).

10. Procédé selon les revendications 8 ou 9, caractérisé en ce que la longueur de l'extrémité (15a) du segment de ruban (15), qui est non scellée et qui dépasse de l'extrémité extérieure (11a) du segment de bande enroulé (11), est comprise entre 10 et 50 mm.

11. Procédé selon une des revendications 8 à 10, caractérisé par l'utilisation d'un ruban thermoplastique perméable aux liquides (32) qui est plus étroit que la bande ouatée (30) et qui s'étend depuis le bord longitudinal (11b) de la bande (30) formant l'extrémité de retrait (10c) du tampon (10) seulement jusqu'au voisinage du bord longitudinal (11c) de la bande (30), qui forme l'extrémité d'introduction (10a) du tampon (10).

12. Procédé selon la revendication 11, caractérisé en ce que l'extrémité d'introduction (10a) du tampon (10) qui n'est pas recouverte par le segment de ruban thermoplastique (15) est profilée en forme de calotte sphérique après la compression radiale de l'ébauche de tampon (12).

13. Procédé selon la revendication 7, caractérisé en ce qu'on utilise une fibre à deux composants comprenant une âme en polyester et une enveloppe en polyéthylène à haute pression.

14. Machine de fabrication du tampon selon une des revendications 1 à 6 en appliquant le procédé selon une des revendications 7 à 13, se composant des dispositifs suivants, placés l'un après l'autre dans la direction de mise en oeuvre :

- une bobine de stockage (50) d'une bande ouatée continue (30) en fibres naturelles et/ou synthétiques ;

- un poste d'affaiblissement (51) de la bande ouatée (30) ;

- un dispositif de transport (55, 56) de la bande ouatée (30) ;

- un dispositif de sectionnement (80) pour former de façon continue les segments (11) de la bande non ouatée ;

- un dispositif d'amenée et de nouage (90) d'un cordon de retrait ;

- un poste d'enroulement (100) servant à l'enroulement du segment de bande (11) lors de la formation d'une ébauche de tampon (12) ;

- une pince de transport et de séparation (110), déplaçable alternativement à peu près dans le plan de transport de la bande ouatée (30) et servant à la séparation de chaque segment de bande (11), et

- un dispositif pour transférer l'ébauche de tampon (12) jusqu'à une presse dans laquelle l'ébauche de tampon (12) peut être comprimée sensiblement radialement à la forme finale du tampon (10), et

- un dispositif de profilage de l'extrémité d'introduction (10a) du tampon,

- caractérisée en ce qu'il est prévu un rouleau de stockage (66) pour un ruban continu (32) formé d'une matière thermoplastique non tissée et perméable aux liquides et d'une largeur plus petite que celle de la bande ouatée (30), ce ruban continu passant dans un poste de découpage (70) de façon à être tronçonné en segments (15) d'une longueur déterminée et il est prévu à la suite du poste (70) un dispositif de séparation et de transfert (82) des segments de ruban (15), qui est lui-même suivi par un poste de scellement (60) servant à sceller le segment de ruban thermoplastique (15) sur le côté extérieur de la bande ouatée (30), et ensuite un dispositif pour le transport continu de la bande ouatée (30) sur laquelle a été scellé le segment de ruban (15) jusqu'au dispositif de séparation de bande (80), et en ce que le poste d'enroulement (100) est associé à un dispositif de soudage et lissage, pouvant être chauffé et servant à appliquer l'extrémité (15a) du segment de ruban (15), s'étendant librement et de façon non scellée sur l'extrémité arrière (11a) du segment de bande (11), contre le segment de ruban (15) scellé sur le segment de bande (11).

15. Machine selon la revendication 14, caractérisée en ce que le poste de découpage (70) du ruban thermoplastique et perméable aux liquides (32) se compose d'une paire de cylindres de coupe (69, 69a), dont le cylindre de découpage (69) est pourvu, sur une partie importante d'une ligne périphérique, d'arêtes de coupe pour effectuer le sectionnement du ruban (32), les cylindres de coupe (69, 69a) étant disposés respectivement au-dessus et en dessous de la voie de déplacement (x) de la bande ouatée (30) et pouvant être entraînés à une vitesse périphérique qui est à peu près égale à la moitié de la vitesse de transport de la bande (30).

16. Machine selon la revendication 14, caractérisée en ce que le dispositif de séparation et transfert (82) du segment de ruban thermoplastique et perméable aux liquides (15) est un rouleau à vide (71), dans le volume intérieur creux duquel est prévu un tiroir (219), qui peut être relié à une source de vide et dont les orifices d'aspiration (220) peuvent être reliés à des orifices d'aspiration (221) du rouleau à vide (71), tandis qu'il est prévu un cylindre de séparation et d'accélération (72) pour le segment de ruban (15), placé sur la périphérie du rouleau à vide (71) à une distance du poste de coupe (70) qui est supérieure à la longueur d'un segment de ruban (15), aussi bien le rouleau à vide (71) que le cylindre d'accélération (72) pouvant être entraînés à une vitesse périphérique qui correspond à la vitesse de transport de la bande ouatée (30).

17. Machine selon la revendication 14, caractérisée en ce que le poste de scellement (60) est composé d'un rouleau de pression (57) placé sur le côté inférieur de la voie de déplacement de la bande (30), et d'un rouleau de scellement (62 ; 201) placé sur le côté supérieur de cette bande, lesdits rouleaux pouvant être chauffés.

18. Machine selon la revendication 15, caractérisée en ce que le rouleau de scellement (62) constitue un rouleau de renvoi pour une bande de scellement sans fin (65), dont la matière résiste à la température de scellement et qui est guidée autour d'autres rouleaux de renvoi (63, 64).

19. Machine selon la revendication 17, caractérisée en ce que le rouleau de pression (57) est déplaçable par intermittence pour le soudage du segment de ruban thermoplastique (15) entre une position de repos et une position d'application contre le rouleau de scellement (62), sous l'action d'un dispositif de commande.

20. Machine selon la revendication 17, caractérisée en ce que le rouleau de scellement (62 ; 201) est associé à au moins un élément transporteur sans fin (73 ; 205) en dessous de la voie de déplacement de la bande ouatée (30).

21. Machine selon la revendication 20, caractérisée en ce qu'un rouleau de renvoi (75) pour une bande transporteuse sans fin (73) est engagé dans un volume intermédiaire existant entre les deux rouleaux de renvoi mutuellement opposés (63, 64) pour la bande de scellement (65).

22. Machine selon une des revendications 17 à 21, caractérisée en ce que deux mâchoires de serrage pouvant être chauffées (101) sont déplaçables alternativement sensiblement radialement par rapport à l'axe du mandrin d'enroulement (33) au-dessus et en dessous de celui-ci à l'aide d'un dispositif de commande de telle sorte que l'extrémité libre (15a) d'un segment de ruban thermoplastique (15) puisse être soudée sur l'ébauche de tampon (12).

23. Machine selon la revendication 17, caractérisée en ce que la périphérie du rouleau de scellement (201) est pourvue d'éléments de scellement pouvant être chauffés (223, 224) ainsi que d'éléments isolants ne pouvant pas être chauffés (225, 226), qui sont répartis en alternance dans la direction périphérique du rouleau de scellement (201).

24. Machine selon la revendication 23, caractérisée en ce que la longueur d'arc des éléments de scellement (223, 224) correspond à peu près à la longueur de la partie du segment de ruban (15) à sceller sur la bande ouatée (30).

25. Machine selon les revendications 17 et 20, caractérisée en ce qu'il est prévu après le rouleau de scellement (201) et au-dessus de la voie de déplacement de la bande ouatée (30) un rouleau de pression (207) auquel est associé un rouleau d'appui (206) en dessous du brin supérieur d'un transporteur à courroies (202), qui est disposé en dessous de la voie de déplacement de la bande ouatée (30).

26. Machine selon la revendication 25, caractérisée en ce qu'il est prévu en arrière du rouleau de pression (207) et au-dessus de la voie de déplacement de la bande ouatée (30), un dispositif de guidage (209) pour cette bande (30).

27. Machine selon les revendications 25 et 26, caractérisée en ce que le dispositif de guidage (209) se compose de plusieurs courroies sans fin (212), qui sont guidées autour de poulies de renvoi (210, 211) dans le même plan que celui où sont disposés les éléments transporteurs sans fin (205) du transporteur à courroies (202), les brins de transport du dispositif de guidage (209) et du transporteur à courroies (202) formant sur le côté de sortie, au moyen de rouleaux de renvoi (208, 227), un intervalle (228) s'élargissant pour permettre l'engagement de la pince de séparation et transport (110) pour saisir l'extrémité avant de la bande ouatée (30).

28. Machine selon les revendications 25 à 27, caractérisée en ce que les poulies de renvoi (211, 204), situées avant le poste de mise en place et nouage (90) de cordon de retrait, du dispositif de guidage (209) et du transporteur à courroies (202) sont respectivement pourvues de segments de guidage (213, 214) faisant saillie radialement vers l'extérieur, qui s'étendent respectivement sur une partie de la périphérie des poulies de renvoi (211, 204) et qui coopèrent pour guider la bande (30) ou le segment de bande (11).

29. Machine selon la revendication 14, caractérisée en ce qu'il est prévu à une certaine distance radiale au-dessus et en dessous du mandrin d'enroulement (33) des mâchoires de serrage (215, 216) de l'extrémité libre (15a) d'un segment de ruban (15), parmi lesquelles la mâchoire supérieure de serrage (216) est disposée symétriquement par rapport à un plan principal vertical passant par l'axe du mandrin d'enroulement tandis que la mâchoire inférieure de serrage (215) s'étend pour l'essentiel dans le troisième quadrant d'un cercle, sur un angle périphérique allant de 180° à 270°.

30. Machine selon la revendication 29, caractérisée en ce que la mâchoire inférieure de serrage (215) est pourvue d'un évidement (217) au travers duquel un élément de scellement (218) pouvant être chauffé peut se déplacer alternativement et à peu près radialement par rapport au mandrin de serrage (33) sous l'action d'un dispositif de commande de telle sorte que l'extrémité libre (15a) du segment de ruban (15) puisse être scellée au moyen dudit élément le long d'une ligne périphérique de l'ébauche de tampon (12).

31. Machine selon la revendication 14, caractérisée en ce que le poste d'affaiblissement (51) de la bande ouatée (30) est placé en amont du poste de scellement (60) et se compose d'une paire de cylindres de perforation et de pincement (53) ainsi que d'une paire de cylindres d'étirage (54), qui sont disposés l'un derrière l'autre dans la direction de mouvement (x) de la bande ouatée (30).

32. Machine selon une des revendications 14 et 31, caractérisée en ce qu'il est prévu après le poste d'affaiblissement (51) une bande porteuse sans fin (56) pour la bande ouatée (30), le rouleau d'entraînement et de tension (56a), placé en avant en considérant la direction de transport (x) de la bande ouatée (30), de la bande transporteuse (56) étant associé à un cylindre de transport (55) qui s'engage sur le côté supérieur de la bande ouatée (30).

33. Machine selon la revendication 14, caractérisée en ce que le dispositif (80) de séparation de la bande ouatée se compose d'une paire de mâchoires pivotantes de pincement (81), qui sont disposées respectivement au-dessus et en dessous de la voie de déplacement (x) de la bande ouatée (30) avant le dispositif de mise en place et nouage (90) du cordon de retrait et qui coopèrent avec la pince de séparation et transport (110), pour séparer un segment de bande (11) de la bande ouatée (30).

FIG. 1

31

31

30

FIG. 2

15a

15

15a

15

30

31

31

31

EP 0 149 155 B1

FIG. 3

FIG. 4

FIG. 5

FIG. 6

3

FIG. 7

EP 0 149 155 B1

FIG. 9

FIG. 8